# EUROPÄISCHE PATENTSCHRIFT

(11) **EP 1 427 725 B1**
(45) Veröffentlichungstag und Bekanntmachung des Hinweises auf die Patenterteilung: **23.03.2005**
(21) Anmeldenummer: 02797941.8
(22) Anmeldetag: 31.08.2002
(51) Int. Cl.: C07D 409/12, A01N 43/56

(54) **PYRAZOLYLSUBSTITUIERTE THIENYLOXY-PYRIDINE**
PYRAZOLYL-SUBSTITUTED THIENYLOXYPYRIDINES
THIENYLOXY-PYRIDINES SUBSTITUEES PAR PYRAZOLYLE

(30) Priorität: 07.09.2001 DE 10144185
(43) Veröffentlichungstag der Anmeldung: 16.06.2004
(73) Patentinhaber: BASF AKTIENGESELLSCHAFT, 67056 Ludwigshafen (DE)
(72) Erfinder: HOFMANN, Michael, 67059 Ludwigshafen (DE); PARRA RAPADO, Liliana, 68161 Mannheim (DE); VON DEYN, Wolfgang, 67435 Neustadt (DE); BAUMANN, Ernst, 67373 Dudenhofen (DE); KORDES, Markus, 67227 Frankenthal (DE); MISSLITZ, Ulf, 67433 Neustadt (DE); WITSCHEL, Matthias, 67098 Bad Dürkheim (DE); ZAGAR, Cyrill, 68167 Mannheim (DE); LANDES, Andreas, 67354 Römerberg Heiligenstein (DE)
(86) Internationale Anmeldenummer: PCT/EP2002/009750
(87) Internationale Veröffentlichungsnummer: WO 2003/022843

(56) Entgegenhaltungen:
- EP-A- 0 887 343
- EP-A- 1 101 764
- WO-A-99/24427
- US-A- 5 851 952

## Beschreibung

Die vorliegende Erfindung betrifft pyrazolylsubstituierte Thienyloxy-Pyridine der Formel I 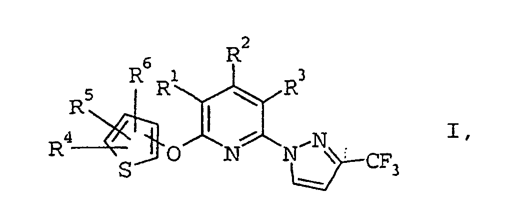 in der die Variablen die folgenden Bedeutungen haben:
- R¹, R³: Wasserstoff, Halogen, Cyano, Nitro, C₁-C₆-Alkyl, C₁-C₆-Halogenalkyl, C₁-C₆-Alkoxy oder C₁-C₆-Halogen-alkoxy;
- R²: Wasserstoff, Halogen, Cyano, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₃-C₆-Alkenyloxy, C₃-C₆-Alkinyloxy, C₁-C₆-Halogenalkoxy, C₁-C₆-Alkoxy-C₁-C₄-alkyl, C₁-C₆-Alkylamino, Di-(C₁-C₄-alkyl)amino, C₁-C₆-Alkylthio, C₁-C₆-Halogenalkylthio, C₁-C₆-Alkyl-sulfinyl, C₁-C₆-Halogenalkylsulfinyl, C₁-C₆-Alkyl-sulfonyl, C₁-C₆-Halogenalkylsulfonyl oder COR⁷;
- R⁴, R⁵, R⁶: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkyl-sulfonyl oder C₁-C₆-Halogenalkylsulfonyl;
- R⁷: Wasserstoff, Hydroxy, C₁-C₆-Alkyl, C₁-C₆-Alkoxy, Amino, C₁-C₆-Alkylamino oder Di-(C₁-C₄-alkyl)amino;
sowie deren landwirtschaftlich brauchbaren Salze.

Außerdem betrifft die Erfindung Zwischenprodukte und Verfahren zur Herstellung von Verbindungen der Formel I, Mittel, welche diese enthalten, sowie die Verwendung dieser Derivate oder der diese Derivate enthaltende Mittel zur Schadpflanzenbekämpfung.

Herbizid wirksame Thienyloxyazine und 2-Aryloxy-6-pyrazol-Pyridine sind aus WO 99/24427 und EP-A-1 101 764 bekannt.

Die herbiziden Eigenschaften der bisher bekannten Verbindungen bzw. die Verträglichkeiten gegenüber Kulturpflanzen können jedoch nur bedingt befriedigen. Es lag daher dieser Erfindung die Aufgabe zugrunde, insbesondere herbizid wirksame Verbindungen mit verbesserten Eigenschaften zu finden.

Demgemäß wurden die pyrazolylsubstituierten Thienyloxy-Pyridine der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di (2-hydroxyeth-1-yl) ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R⁷ genannten organischen Molekülteile stellen Sammelbegriffe für individuelle Aufzählungen der einzelnen Gruppenmitglieder dar. Sämtliche Kohlenwasserstoffketten, also alle Alkyl-, Alkenyl, Alkinyl, Halogenalkyl-, Halogenalkenyl, Halogenalkinyl, Alkoxy-, Alkenyloxy, Alkinyloxy, Halogenalkoxy-, Alkoxyalkyl, Alkiylamino, Dialkylamino, Alkylthio-, Halogenalkylthio-, Alkylsulfinyl-, Halogenalkylsulfinyl Alkylsulfonyl- und Halogenalkylsulfonyl-Teile können geradkettig oder verzweigt sein. Sofern nicht anders angegeben tragen halogenierte Substituenten vorzugsweise ein bis fünf, insbesondere ein bis drei, gleiche oder verschiedene Halogenatome. Die Bedeutung Halogen steht jeweils für Fluor, Chlor, Brom oder Iod.

Ferner bedeuten beispielsweise:
◆ C₁-C₄-Alkyl sowie die Alkylteile von Hydroxy-C₁-C₄-alkyl, Hydroxy-C₁-C₄-alkoxy-C₁-C₄-alkyl, Tri(C₁-C₄-alkyl)sulfonium und Tri(C₁-C₄-alkyl)sulfoxonium: z.B. Methyl, Ethyl, 1-Propyl, 1-Methylethyl, 1-Butyl, 1-Methylpropyl, 2-Methylpropyl und 1,1-Dimethylethyl;
◆ C₁-C₆-Alkyl: C₁-C₄-Alkyl, wie voranstehend genannt, sowie z.B. 1-Pentyl, 1-Methylbutyl, 2-Methylbutyl, 3-Methylbutyl, 2,2-Dimethylpropyl, 1-Ethylpropyl, 1-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
◆ C₂-C₆-Alkenyl: z.B. Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 3-Methyl-1-pentenyl, 3-Methyl-1-pentenyl, 4-Methyl-1-pentenyl, 1-Methyl-2-pentenyl, 2-Methyl-2-pentenyl, 3,-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
◆ C₂-C₆-Alkinyl: z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
◆ C₁-C₆-Halogenalkyl: einen C₁-C₆-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4,-Fluorbutyl, 4-Chlorbutyl, 4-Brombutyl, Nonafluorbutyl, 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
◆ C₂-C₆-Halogenalkenyl: ein C₂-C₆-Alkenylrest, wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromvinyl, 2-Bromallyl, 3-Bromallyl, 2,3-Dibromallyl, 3,3-Dibromallyl, 2,3,3-Tribromallyl oder 2,3-Dibrombut-2-enyl;
◆ C₂-C₆-Halogenalkinyl: ein C₂-C₆-Alkinylrest, wie voranstehend genannt, der partiell oder vollständig durch Fluor, Chlor, Brom und/oder Iod substituiert ist, z.B. 1,1-Difluorprop-2-in-1-yl, 3-Iod-prop-2-in-1-yl, 4-Fluorbut-2-in-1-yl, 4-Chlorbut-2in-1-yl, 1,1-Difluorbut-2-in-1-yl, 4-Iodbut-3-in-1-yl, 5-Fluorpent-3-in-1-yl, 5-Iodpent-4-in-1-yl, 6-Fluorhex-4-in-1-yl oder 6-Iodhex-5-in-1-yl;
◆ C₁-C₄-Alkoxy: sowie die Alkoxyteile von Hydroxy-C₁-C₄-alkoxy-C₁-C₄-alkyl, z.B. Methoxy, Ethoxy, Propoxy, 1-Methylethoxy, Butoxy, 1-Methylpropoxy, 2-Methylpropoxy und 1,1-Dimethylethoxy;
◆ C₁-C₆-Alkoxy: C₁-C₄-Alkoxy wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy,1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
◆ C₃-C₆-Alkenyloxy: z.B. Prop-1-en-1-yloxy, Prop-2-en-1-yloxy, 2-Methylethenyloxy, Buten-1-yloxy, Buten-2-yloxy, Buten-3-yloxy, 1-Methyl-prop-1-en-1-yloxy, 2-Methyl-prop-1-en-1-yloxy, 1-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methylbut-3-en-1-yloxy, 2-Methyl-but-3-en-1-yloxy, 3-Methylbut-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloay, 1-Methyl-pent-2-en-1-yloxy, 2-Methyl-pent-2-en-1-yloxy, 3-Methyl-pent-2-en-1-yloxy, 4-Methylpent-2-en-1-yloxy, 1-Methyl-pent-3-en-1-yloxy, 2-Methylpent-3-en-1-yloxy, 3-Methyl-pent-3-en-1-yloxy, 4-Methylpent-3-en-2-yloxy, 1-Methyl-pent-4-en-1-yloxy, 2-Methylpent-4-en-1-yloxy, 3-Methyl-pent-4-en-1-yloxy, 4-Methylpent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 3,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethylbut-1-en-1-yloxy, 2-Ethyl-but-2-en-1-yloxy, 2-Ethylbut-3-en-1-yloxy, 1,1,2-Trimethyl-prop-2-en-1-yloxy, 1-Ethyl-1-methyl-prop-2-en-1-ylox-y, 1-Ethyl-2-methylprop-1-en-1-yloxy und 1-Ethyl-2-methyl-prop-2-en-1-yloxy;
◆ C₃-C₆-Alkinyloxy: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy, Pent-2-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methylpent-1-in-3-yloxy, 3-Methyl-pent-1-in-4-yloxy, 3-Methylpent-1-in-5-yloxy, 4-Methyl-pent-1-in-1-yloxy, 4-Methylpent-2-in-4-yloxy und 4-Methylpent-2-in-5-yloxy;
◆ C₁-C₆-Halogenalkoxy: einen C₁-C₆-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy, Nonafluorbutoxy, 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy und Dodecafluorhexoxy;
◆ C₁-C₆-Alkoxy-C₁-C₄-alkyl: durch C₁-C₆-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy) methyl, (1,1-Dimethylethoxy)methyl, 2-(Methoxy)ethyl, 2-(Ethoxy)ethyl, 2- (Propoxy) ethyl, 2-(1-Methylethoxy) ethyl, 2-(Butoxy)ethyl, 2-(1-Methylpropoxy)ethyl, 2-(2-Methylpropoxy) ethyl, 2-(1,1-Dimethylethoxy)ethyl, 2-(Methoxy)-propyl, 2-(Ethoxy)propyl, 2-(Propoxy)propyl, 2-(1-Methylethox-y)propyl, 2-(Butoxy)propyl, 2-(1-Methylpropoxy)propyl, 2- (2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy) propyl, 3-(1,1-Dimethylethoxy)propyl, 2-(Methoxy)-butyl, 2-(Ethoxy) butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy) butyl, 3-(1,1-Dimethylethoxy)butyl, 4-(Methoxy)butyl, 4- (Ethoxy) butyl , 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)butyl;
◆ C₁-C₆-Alkylamino:z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
◆ Di-(C₁-C₄-alkyl)-amino: z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N- (1,1-Dimethylethyl) -N-methylamino, N-Ethyl-N-propylamino, N-Ethyl-N- (1-methylethyl) amino, N-Butyl-N-ethylamino, N-Ethyl-N-(1-methylpropyl)amino, N-Ethyl-N-(2-methylpropyl) amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N- (1-methylethyl) amino, N-(1-Methylethyl)-N-(1-methylpropyl)amino, N-(2-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
◆ C₁-C₆-Alkylthio: z.B. Methylthio, Ethylthio, Propylthio, 1-Methylethylthio, Butylthio, 1-Methylpropylthio, 2-Methylpropylthio und 1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio;
◆ C₁-C₆-Halogenalkylthio: einen C₁-C₆-Alkylthiorest wie voranstehend genannt, der partiell oder vollständig durch Fluor, Chlor, Brom und/oder Iod substituiert ist, also z.B. Fluormethylthio, Difluormethylthio, Trifluormethylthio, Chlor-difluormethylthio, Bromdifluormethylthio, 2-Fluorethylthio, 2-Chlorethylthio, 2-Bromethylthio, 2-Iodethylthio, 2,2 -Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2- chlorethylthio, 1-(Brommethyl)-2-bromethylthio, 4-Fluorbutylthio, 4-Chlorbutylthio, 4-Brombutylthio, Nonafluorbutylthio, 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio und Dodecafluorhexylthio;
◆ C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
◆ C₁-C₆-Halogenalkylsulfinyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl und Dodecafluorhexylsulfinyl;
◆ C₁-C₆-Alkylsulfonyl (C₁-C₆-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
◆ C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl) -2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;

In einer besonderen Ausführungsform haben die Variablen der Verbindungen der Formel I folgende Bedeutungen, wobei diese für sich allein betrachtet als auch in der Kombination miteinander besondere Ausgestaltungen der Verbindungen der Formel I darstellen:

Bevorzugt sind die pyrazolylsubstituierten Thienyloxy-Pyridine der Formel I, in der
- R¹, R³: Wasserstoff, Halogen, Cyano, Nitro, C₁-C₆-Alkyl, C₁-C₆-Halogenalkyl;
besonders bevorzugt Wasserstoff, Halogen wie Fluor, Chlor oder Brom, C₁-C₆-Alkyl, wie Methyl oder Ethyl;
insbesondere bevorzugt Wasserstoff, Fluor, Chlor oder Methyl;
bedeuten.

Außerdem sind die pyrazolylsubstituierten Thienyloxy-Pyridine der Formel I bevorzugt, in der
- R¹: Wasserstoff; und
- R³: Wasserstoff, Halogen, Cyano, Nitro, C₁-C₆-Alkyl oder C₁-C₆-Halogenalkyl;
besonders bevorzugt Wasserstoff, Halogen wie Fluor, Chlor oder Brom, C₁-C₆-Alkyl, wie Methyl oder Ethyl;
insbesondere bevorzugt Wasserstoff, Fluor, Chlor oder Methyl;
bedeuten.

Außerdem sind die pyrazolylsubstituierten Thienyloxy-Pyridine der Formel I bevorzugt, in der
- R¹: Wasserstoff, Halogen, Cyano, Nitro, C₁-C₆-Alkyl oder C₁-C₆-Halogenalkyl ;
besonders bevorzugt Wasserstoff, Halogen wie Fluor, Chlor oder Brom, C₁-C₆-Alkyl, wie Methyl oder Ethyl;
insbesondere bevorzugt Wasserstoff, Fluor, Chlor oder Methyl; und
- R³: Wasserstoff;
bedeuten.

Ebenso bevorzugt sind die pyrazolylsubstituierten Thienyloxy-Pyridine der Formel I, in der
- R²: Wasserstoff, Halogen, Cyano, C₁-C₆-Halogenalkyl, C₁-C₆-Alkoxy, C₁-C₆-Alkylamino, Di (C₁-C₄-alkyl) amino, C₁-C₆-Alkylthio oder COR⁷;
besonders bevorzugt Wasserstoff, Halogen, wie z.B. Fluor, Chlor oder Brom, Cyano oder C₁-C₆-Halogenalkyl wie z.B. Fluormethyl, Chlormethyl, Brommethyl oder Trifluormethyl, C₁-C₆-Alkoxy wie z.B. Methoxy oder C₁-C₆-Alkylthio, wie z.B. Methylthio;
insbesondere bevorzugt Wasserstoff, Fluor, Chlor, Cyano, Methoxy oder Trifluormethyl
bedeutet.

Des weiteren werden die pyrazolylsubstituierten Thienyloxy-Pyridine der Formel I bevorzugt, in der
- R²: Wasserstoff, Halogen, Cyano, C₁-C₆-Alkoxy, C₁-C₆-Alkenyloxy, C₁-C₆-Alkinyloxy, C₁-C₆-Halogenalkoxy, C₁-C₆-Alkylamino, Di (C₁-C₄-alkyl) amino, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
sehr bevorzugt Wasserstoff, Halogen, Cyano, C₁-C₆-Alkoxy, C₁-C₆-Alkylamino, Di(C₁-C₄-alkyl)amino oder C₁-C₆-Alkylthio;
besonders bevorzugt Wasserstoff, Halogen, wie z.B. Fluor, Chlor oder Brom, Cyano, C₁-C₆-Alkoxy wie z.B. Methoxy oder C₁-C₆-Alkylthio, wie z.B. Methylthio;
insbesondere bevorzugt Wasserstoff, Fluor, Chlor, Cyano oder Methoxy;
bedeutet.

Ebenso bevorzugt sind die pyrazolylsubstituierten Thienyloxy-Pyridine der Formel I, in der
- R²: C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy-C₁-C₄-alkyl oder COR⁷;
sehr bevorzugt C₁-C₆-Halogenalkyl oder COR⁷;
besonders bevorzugt C₁-C₆-Halogenalkyl wie z.B. Fluormethyl, Chlormethyl, Brommethyl oder Trifluormethyl;
insbesondere bevorzugt Trifluormethyl;
bedeutet.

Des weiteren werden die pyrazolylsubstituierten Thienyloxy-Pyridine der Formel I bevorzugt, in der
- R¹, R³: Wasserstoff, Halogen, Cyano, Nitro, C₁-C₆-Alkyl oder C₁-C₆-Halogenalkyl ;
besonders bevorzugt Wasserstoff, Halogen wie Fluor, Chlor oder Brom, C₁-C₆-Alkyl, wie Methyl oder Ethyl, C₁-C₆-Halogenalkyl wie Fluormethyl, Chlormethyl oder Trifluormethyl;
insbesondere bevorzugt Wasserstoff, Fluor, Chlor oder Methyl; und
- R²: Wasserstoff, Halogen, Cyano, C₁-C₆-Halogenalkyl, C₁-C₆-Alkoxy, C₁-C₆-Alkylamino, Di(C₁-C₄-alkyl)amino, C₁-C₆-Alkylthio oder COR⁷;
besonders bevorzugt Wasserstoff, Halogen, wie z.B. Fluor, Chlor oder Brom, Cyano, C₁-C₆-Halogenalkyl wie z.B. Fluormethyl, Chlormethyl, Brommethyl oder Trifluormethyl, C₁-C₆-Alkoxy wie z.B. Methoxy oder C₁-C₆-Alkylthio, wie z.B. Methylthio;
insbesondere bevorzugt Wasserstoff, Fluor, Chlor, Cyano, Methoxy oder Trifluormethyl;
bedeuten.

Ebenso sind die pyrazolylsubstituierten Thienyloxy-Pyridine der Formel I bevorzugt, in der
- R¹: Wasserstoff, Halogen, Cyano, Nitro, C₁-C₆-alkyl oder C₁-C₆-Halogenalkyl;
besonders bevorzugt Wasserstoff, Halogen wie Fluor, Chlor oder Brom, C₁-C₆-Alkyl, wie Methyl oder Ethyl, C₁-C₆-Halogenalkyl wie Fluormethyl, Chlormethyl oder Trifluormethyl;
insbesondere bevorzugt Wasserstoff, Fluor, Chlor oder Methyl; und
- R²: Wasserstoff, Halogen, Cyano, C₁-C₆-Halogenalkyl, C₁-C₆-Alkoxy, C₁-C₅-Alkylamino, Di(C₁-C₄-alkyl)amino, C₁-C₆-Alkylthio oder COR⁷;
besonders bevorzugt Wasserstoff, Halogen, wie z.B. Fluor, Chlor oder Brom, Cyano, C₁-C₆-Halogenalkyl wie z.B. Fluormethyl, Chlormethyl, Brommethyl oder Trifluormethyl, C₁-C₆-Alkoxy wie z.B. Methoxy oder C₁-C₆-Alkylthio, wie z.B. Methylthio;
insbesondere bevorzugt Wasserstoff, Fluor, Chlor, Cyano, Methoxy oder Trifluormethyl; und
- R³: Wasserstoff
bedeuten.

Daneben sind die pyrazolylsubstituierten Thienyloxy-Pyridine der Formel I bevorzugt, in der
- R¹: Wasserstoff;
- R²: Wasserstoff, Halogen, Cyano, C₁-C₆-Halogenalkyl, C₁-C₆-Alkoxy, C₁-C₆-Alkylamino, Di(C₁-C₄-alkyl) amino, C₁-C₆-Alkylthio oder COR⁷;
besonders bevorzugt Wasserstoff, Halogen, wie z.B. Fluor, Chlor oder Brom, Cyano, C₁-C₆-Halogenalkyl wie z.B. Fluormethyl, Chlormethyl, Brommethyl oder Trifluormethyl, C₁-C₆-Alkoxy wie z.B. Methoxy oder C₁-C₆-Alkylthio, wie z.B. Methylthio;
insbesondere bevorzugt Wasserstoff, Fluor, Chlor, Cyano, Methoxy oder Trifluormethyl; und
- R³: Wasserstoff;
bedeuten.

Des weiteren sind die pyrazolylsubstituierten Thienyloxy-Pyridine der Formel I bevorzugt, in der
- R¹: Halogen, wie z. Fluor, Chlor oder Brom;
besonders bevorzugt Fluor oder Chlor;
- R²: Wasserstoff, Halogen, Cyano, C₁-C₆-Halogenalkyl, C₁-C₆-Alkoxy, C₁-C₆-Alkylamino, Di(C₁-C₄-alkyl)amino, C₁-C₆-Alkylthio oder COR⁷;
besonders bevorzugt Wasserstoff, Halogen, wie z.B. Fluor, Chlor oder Brom, Cyano, C₁-C₆-Halogenalkyl wie z.B. Fluormethyl, Chlormethyl, Brommethyl oder Trifluormethyl, C₁-C₆-Alkoxy wie z.B. Methoxy oder C₁-C₆-Alkylthio, wie z.B. Methylthio;
insbesondere bevorzugt Wasserstoff, Fluor, Chlor, Cyano, Methoxy oder Trifluormethyl; und
- R³: Halogen, wie z. Fluor, Chlor oder Brom;
besonders bevorzugt Fluor oder Chlor;
bedeuten.

Ebenso bevorzugt sind die pyrazolylsubstituierten Thienyloxy-Pyridine der Formel I, in der jeweils unabhängig voneinander
- R⁴, R⁵, R⁶: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl, C₁-C₆-Halogenalkylsufonyl;
besonders bevorzugt Wasserstoff, Halogen, Cyano, C₁-C₆-Alkyl, C₁-C₆-Halogenalkyl, C₁-C₆-Halogenalkoxy, C₁-C₆-Alkylsulfonyl oder C₁-C₆-Halogenalkylsufonyl;
insbesondere bevorzugt Wasserstoff, Halogen, wie Fluor, Chlor oder Brom, C₁-C₆-Halogenalkyl, wie Trifluormethyl, Trichlormethyl oder Difluormethyl, C₁-C₆-Halogenalkoxy wie Difluormethoxy oder Trifluormethoxy;
sehr bevorzugt Wasserstoff, Fluor, Chlor, Trifluormethyl oder Difluormethoxy;
bedeuten.

Ebenso bevorzugt sind die pyrazolylylsubstituierten Thienyloxy-Pyridine der Formel I, in der R⁶ Wasserstoff und jeweils unabhängig voneinander
- R⁴, R⁵: Wasserstoff, Halogen, C₁-C₆-Alkyl oder C₁-C₆-Halogenalkyl;
besonders bevorzugt Wasserstoff, Chlor, Methyl oder Trifluormethyl;
bedeuten.

Ebenso bevorzugt sind die pyrazolylylsubstituierten Thienyloxy-Pyridine der Formel I, in der
- R⁷: Wasserstoff, C₁-C₆-Alkyl oder C₁-C₆-Alkoxy, wie z.B. Methoxy oder Ethoxy,
besonders bevorzugt Wasserstoff, Methoxy oder Ethoxy;
bedeutet.

Insbesondere ebenso bevorzugt sind die pyrazolylsubstituierten Thienyloxy-Pyridine der Formel I, in der der Thienylrest in 3-Position über das Sauerstoffatom mit dem Pyridingerüst verknüpft und mit R⁴ und R⁵ in 4 bzw. 5-Position substituiert ist.

Ebenso insbesondere bevorzugt sind die pyrazolylsubstituierten Thienyloxy-Pyridine der Formel I, in der der Thienylrest in 2-Position über das Sauerstoffatom mit dem Pyridingerüst verknüpft und mit R⁴ und R⁵ in 4 bzw. 5-Position substituiert ist.

Ebenso bevorzugt sind die pyrazolylsubstituierten Thienyloxy-Pyridine der Formel I, in der R⁵ und R⁶ Wasserstoff und
- R⁴: Halogen, Cyano, C₁-C₆-Alkyl, C₁-C₆-Halogenalkyl oder C₁-C₆-Halogenalkoxy;
besonders bevorzugt Halogen oder C₁-C₆-Halogenalkyl; sehr bevorzugt Fluor, Chlor oder Trifluormethyl;
bedeutet.

Insbesondere ebenso bevorzugt sind die pyrazolylsubstituierten Thienyloxy-Pyridine der Formel I, in der der Thienylrest in 3-Position über das Sauerstoffatom mit dem Pyridingerüst verknüpft und mit R⁴ in 5-Position substituiert ist.

Insbesondere ebenso bevorzugt sind die pyrazolylsubstituierten Thienyloxy-Pyridine der Formel I, in der der Thienylrest in 2-Position über das Sauerstoffatom mit dem Pyridingerüst verknüpft und mit R⁴ in 5-Position substituiert ist.

Außerordentlich bevorzugt sind Verbindungen der Formel Ia (mit R⁴ = 5-CF₃, R⁵ = H, R⁶ = H; der Thienylrest ist in 3-Position über ein Sauerstoffatom mit dem Pyridingerüst verknüpft), insbesondere die Verbindungen Ia.1 bis Ia.52 der Tabelle 1, wobei die Definitionen der Variablen R¹ bis R⁶ nicht nur in Kombination miteinander, sondern auch jeweils für sich allein betrachtet für die erfindungsgemäßen Verbindungen eine besondere Rolle spielen.

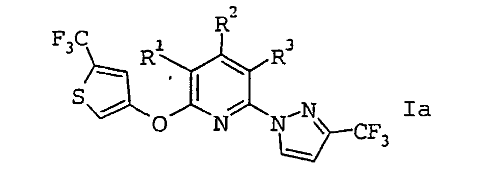

**Tabelle 1**

| Nr. | R¹ | R² | R³ |
|---|---|---|---|
| Ia.1 | H | H | H |
| Ia.2 | H | Cl | H |
| Ia.3 | H | CN | H |
| Ia.4 | H | CH₂Br | H |
| Ia.5 | H | CF₃ | H |
| Ia.6 | H | OCH₃ | H |
| Ia.7 | H | OCH₃ | CN |
| Ia.8 | CN | OCH₃ | H |
| Ia.9 | H | SCH₃ | H |
| Ia.10 | H | CHO | H |
| Ia.11 | H | CO₂CH₃ | H |
| Ia.12 | H | H | Cl |
| Ia.13 | H | H | CN |
| Ia.14 | H | H | NO₂ |
| Ia.15 | H | H | CH₃ |
| Ia.16 | H | H | CH₂Cl |
| Ia.17 | H | H | CF₃ |
| Ia.18 | Cl | H | H |
| Ia.19 | CN | H | H |
| Ia.20 | NO₂ | H | H |
| Ia.21 | CH₃ | H | H |
| Ia.22 | CH₂Cl | H | H |
| Ia.23 | CF₃ | H | H |
| Ia.24 | F | H | F |
| Ia.25 | F | F | F |
| Ia.26 | F | Cl | F |
| Ia.27 | F | Br | F |
| Ia.28 | F | CN | F |
| Ia.29 | F | CF₃ | F |
| Ia.30 | F | OCH₃ | F |
| Ia.31 | F | SCH₃ | F |
| Ia.32 | F | N(CH₃)₂ | F |
| Ia.33 | F | CO₂H | F |
| Ia.34 | F | CO₂CH₃ | F |
| Ia.35 | Cl | H | Cl |
| Ia.36 | Cl | F | Cl |
| Ia.37 | Cl | Cl | Cl |
| Ia.38 | Cl | CN | Cl |
| Ia.39 | Cl | CF₃ | Cl |
| Ia.40 | Cl | OCH₃ | Cl |
| Ia.41 | Cl | SCH₃ | Cl |
| Ia.42 | NO₂ | H | NO₂ |
| Ia.43 | CH₃ | H | CH₃ |
| Ia.44 | CF₃ | H | CF₃ |
| Ia.45 | Cl | H | CF₃ |
| Ia.46 | NO₂ | H | CF₃ |
| Ia.47 | CH₃ | H | CF₃ |
| Ia.48 | CF₃ | H | Cl |
| Ia.49 | CF₃ | H | NO₂ |
| Ia.50 | CF₃ | H | CH₃ |
| Ia.51 | F | H | CN |
| Ia.52 | CN | H | F |

Ebenso außerordentlich bevorzugt sind die Verbindungen der Formel Ib, insbesondere die Verbindungen Ib.1 bis Ib.52, die sich von den entsprechenden Verbindungen Ia.1 bis Ia.52 dadurch unterscheiden, daß R⁴ in 5-Position Chlor ist.

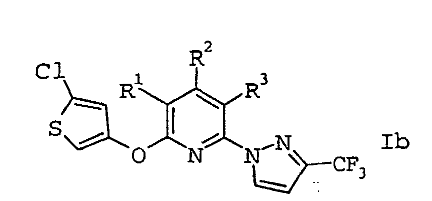

Ebenso außerordentlich bevorzugt sind die Verbindungen der Formel Ic, insbesondere die Verbindungen Ic.1 bis Ic.52, die sich von den entsprechenden Verbindungen Ia.1 bis Ia.52 dadurch unterscheiden, daß der Thienylrest in 2-Position über das Sauerstoffatom mit dem Pyridingerüst verknüpft ist.

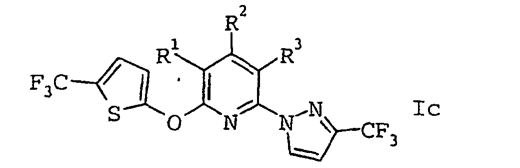

Ebenso außerordentlich bevorzugt sind die Verbindungen der Formel Id, insbesondere die Verbindungen Id.1 bis Id.52, die sich von den entsprechenden Verbindungen Ia.1 bis Ia.52 dadurch unterscheiden, daß R⁴ in 5-Position Chlor ist und der Thienylrest in 2-Position über das Sauerstoffatom mit dem Pyridingerüst verknüpft ist.

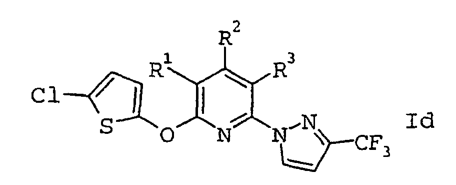

Ebenso außerordentlich bevorzugt sind die Verbindungen der Formel Ie, insbesondere die Verbindungen Ie.1 bis Ie.52, die sich von den entsprechenden Verbindungen Ia.1 bis Ia.52 dadurch unterscheiden, daß R⁴ in 4-Position Trifluormethyl ist und der Thienylrest in 2-Position über das Sauerstoffatom mit dem Pyridingerüst verknüpft ist.

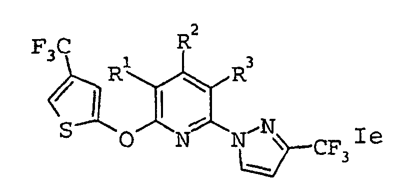

Ebenso außerordentlich bevorzugt sind die Verbindungen der Formel If, insbesondere die Verbindungen If.1 bis If.52, die sich von den entsprechenden Verbindungen Ia.1 bis Ia.52 dadurch unterscheiden, daß R⁴ in 4-Position Chlor ist und der Thienylrest in 2-Position über das Sauerstoffatom mit dem Pyridingerüst verknüpft ist.

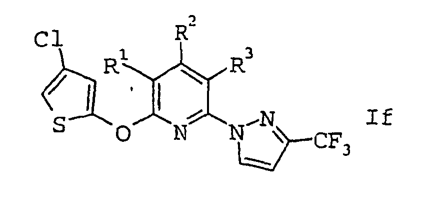

Die pyrazolylsubstituierten Thienyloxy-Pyridine der Formel I sind auf verschiedene Art und Weise erhältlich, beispielsweise nach folgenden Verfahren.

### Verfahren A

Ausgehend von Pyridinen der Formel V erhält man durch Umsetzung mit 3-Trifluormethyl-1H-pyrazol IV die 3-Trifluormethyl-1H-pyrazol-1-yl-substituierten Pyridine der Formel III. L¹ und L² stehen für nucleophil austauschbare Abgangsgruppen wie Halogen, z.B. Fluor, Chlor und Brom, C₁-C₄-Alkylsulfonyl, wie z.B. Methylsulfonyl, C₁-C₄-Alkylsulfonyloxy, wie z.B. Methylsulfonyloxy, C₁-C₄-Halogenalkylsulfonyloxy oder Trialkylammonium, bevorzugt sind Fluor, Chlor oder Brom, C₁-C₄-Alkylsulfonyl, wie z.B. Methylsulfonyl, oder C₁-C₄-Halogenalkylsulfonyloxy, wie z.B. Trifluormethylsulfonyloxy. Diese werden dann mit Hydroxythiophenen der Formel II zu pyrazolsubstituierten Thienyloxy-Pyridinen der Formel I umgesetzt:

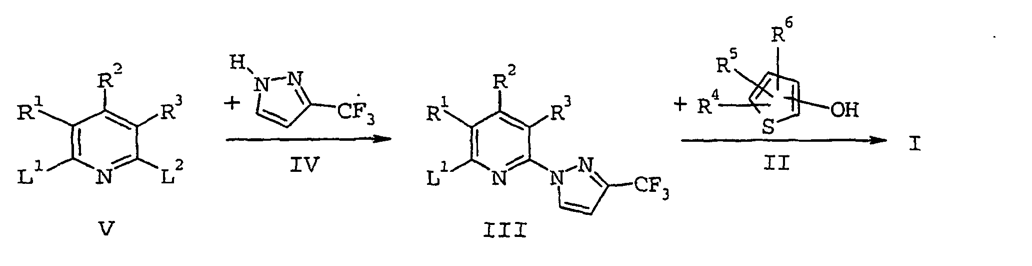

Die Umsetzung von Pyridinen der Formel V zu 3-Trifluormethyl-1Hpyrazol-1-yl-substituierten Pyridinen der Formel III erfolgt üblicherweise bei Temperaturen von 0°C bis 200°C, vorzugsweise 10°C bis 100°C, in einem inerten organischen Lösungsmittel in Gegenwart einer Base [vgl. WO 98/40379; EP 1 101 764].

Geeignete Lösungsmittel sind aliphatische Kohlenwasserstoffe wie Pentan, Hexan, Cyclohexan und Gemische von C₅-C₈-Alkanen, Ether wie Diethylether, Diisopropylether, tert.-Butylmethylether, Dioxan, Anisol und Tetrahydrofuran, Nitrile wie Acetonitril und Propionitril, sowie Dimethylsulfoxid, Dimethylformamid und Dimethylacetamid, besonders bevorzugt Acetonitril und Dimethylformamid.

Ebenfalls geeignete Lösungsmittel sind aromatische Kohlenwasserstoffe wie z.B. Toluol und Xylol.

Es können auch Gemische der genannten Lösungsmittel verwendet werden.

Als Basen kommen im allgemeinen anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetall- und Erdalkalimetallalkoholate wie Natriummethanolat, Natriumethanolat, Kaliumethanolat, Kaliumtert.-Butylat und Kalium-tert.-Pentylat; organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydrid, Kalium-tert.-Butylat und Kalium-tert.-Pentylat.

Ebenso bevorzugt wird als Base Cäsiumcarbonat.

Die Basen werden im allgemeinen in äquimolaren Mengen eingesetzt, sie können aber auch im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, V in einem Überschuß bezogen auf IV einzusetzen.

Es kann von Vorteil sein, katalytische Mengen von Kupfer oder Cu(I)-Salzen, wie z.B. CuBr oder Cu-triflat einzusetzen.

Die Umsetzung von 3-Trifluormethyl-1H-pyrazol-1-yl-substituierten Pyridinen der Formel III zu pyrazolylsubstituierten Thienyloxy-Pyridinen der Formel I erfolgt üblicherweise bei Temperaturen von 50°C bis 200°C, vorzugsweise 50°C bis 150°C, in einem inerten organischen Lösungsmittel in Gegenwart einer Base [vgl. WO 98/40379; EP 1 101 764].

Geeignete Lösungsmittel sind aliphatische Kohlenwasserstoffe wie Pentan, Hexan, Cyclohexan und Gemische von C₅-C₈-Alkanen, Ether wie Diethylether, Diisopropylether, tert.-Butylmethylether, Dioxan, Anisol, Tetrahydrofuran und Diethylenglycoldimethylether, Nitrile wie Acetonitril und Propionitril, sowie Dimethylsulfoxid, Dimethylformamid, Dimethylacetamid, N-Methylpyrrolidon und Sulfolan, besonders bevorzugt Acetonitril, Diethylenglycoldimethylether, Dimethylformamid, N-Methylpyrrolidon und Sulfolan. Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetall- und Erdalkalimetallalkoholate wie Natriummethanolat, Natriumethanolat, Kaliumethanolat, Kaliumtert.-Butylat und Kalium-tert.-Pentylat; organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumcarbonat, Natriumhydrid, Kalium-tert.-Butylat und Kalium-tert.-Pentylat.

Die Basen werden im allgemeinen in äquimolaren Mengen eingesetzt, sie können aber auch im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß bezogen auf III einzusetzen.

Die für die Herstellung der Verbindungen I benötigten Ausgangsstoffe sind in der Literatur bekannt oder können gemäß der zitierten Literatur hergestellt werden [vgl. EP 1 101 764].

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischenund Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden können. Sofern die Zwischen- und Endprodukte als Feststoffe erhalten werden, kann die Reinigung auch durch Umkristallisieren oder Digerieren erfolgen.

### Verfahren B

Ein Dihalogenpyridin der Formel V (mit L¹=Hal und L²=Hal') wird mit Natrium- oder Kaliummercaptan der Formel VIII zu Pyridinen der Formel VII umgesetzt. R^{a} steht dabei für C₁-C₆-Alkyl, bevorzugt Methyl. Die Pyridine der Formel VII können anschließend mit einem Pyrazol der Formel IV zu pyrazolylsubstituierten Pyridinen der Formel VI reagieren:

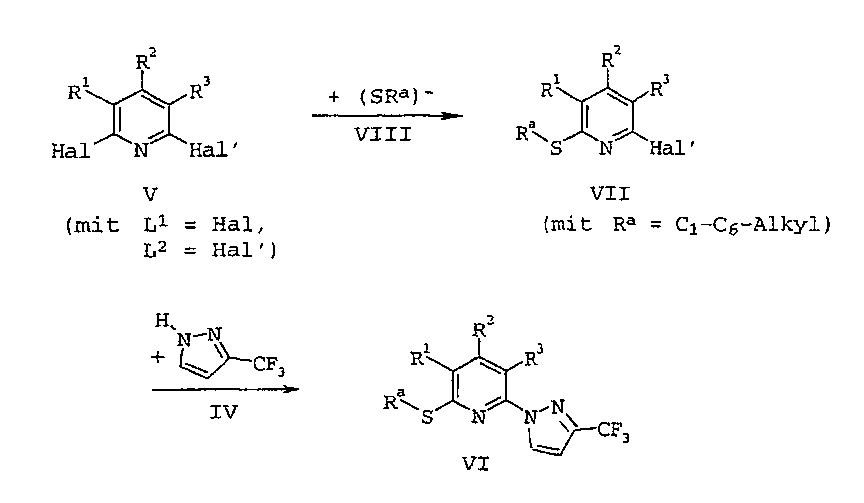

Die Umsetzung zu Pyridinen der Formel VII erfolgt üblicherweise bei Temperaturen von 0°C bis 80°C in einem inerten organischen Lösungsmittel [vgl. WO 98/40379].

Geeignete Lösungsmittel sind Ether wie Diethylether, Diisopropylether, tert.-Butylmethylether, Dioxan, Anisol und Tetrahydrofuran, besonders bevorzugt Tetrahydrofuran.

Es können auch Gemische der genannten Lösungsmittel verwendet werden.

Die Edukte werden im allgemeinen in äquimolaren Mengen miteinander umgesetzt.

Die Aufarbeitung kann in an sich bekannter Weise zum Produkt hin erfolgen.

Die Umsetzung von Pyridinen der Formel VII zu pyrazolylsubstituierten Pyridinen der Formel VI erfolgt üblicherweise bei Temperaturen von 50 °C bis 200 °C, vorzugsweise 50 °C bis 150 °C analog der Umsetzung von V zu III (vgl. Verfahren A).

Anschließend werden die pyrazolylsubstituierten Pyridine der Formel VI zu Verbindungen der Formel III (mit L¹=SO₂R^{a}) oxidiert. Durch die weitere Umsetzung mit Hydroxythiophenen der Formel II erhält man die pyrazolylsubstituierten Thienyloxy-Pyridine der Formel I:

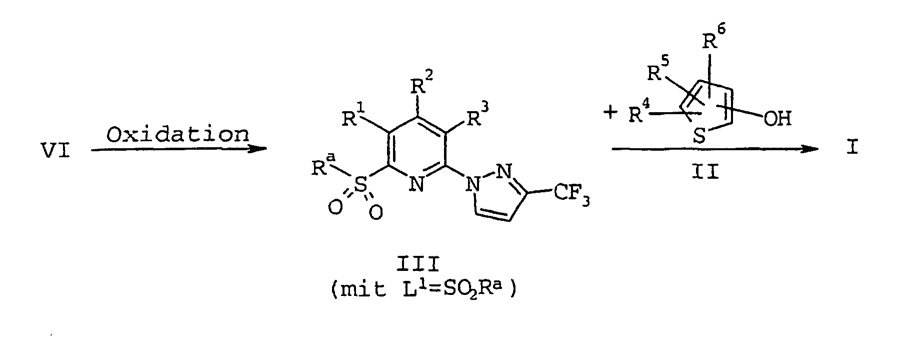

Die Oxidation erfolgt üblicherweise bei Temperaturen von 0°C bis 100°C, vorzugsweise 25°C, in einem inerten organischen Lösungsmittel [vgl. J. March, Organic chemistry, 1992, 1201-1203.].

Geeignete Oxidationsmittel sind z.B. Metachlorperbenzoesäure, Peroxyessigsäure, Trifluorperoxyessigsäure, Wasserstoffperoxid, Natriumperiodat oder Oxon®. Es kann von Vorteil sein, die Reaktion in Gegenwart eines Katalysators, z.B. Natriumwolframat, durchzuführen.

Geeignete Lösungsmittel sind halogenierte Kohlenwasserstoffe wie Methylenchlorid, Chloroform und Chlorbenzol, Alkohole wie Methanol, Ethanol, n-Propanol, Isopropanol, n-Butanol und tert.-Butanol.

Die Edukte werden im allgemeinen in äquimolaren Mengen miteinander umgesetzt. Es kann für die Ausbeute vorteilhaft sein, das Oxidationsmittel in einem Überschuß bezogen auf VI einzusetzen.

Die Aufarbeitung kann in an sich bekannter Weise zum Produkt hin erfolgen.

Die Umsetzung von Verbindungen der Formel III mit Hydroxythiophenen der Formel II erfolgt unter den gleichen Bedingungen wie die Umsetzung von III zu I (vgl. Verfahren A).

### Verfahren C

Es ist ebenso möglich den Stickstoffheterocyclus direkt aus einem entsprechenden Aminopyridin aufzubauen. Man erhält dann pyrazolylsubstituierte Pyridine, welche anschließend gemäß der zuvor dargestellten Reaktionen weiter modifiziert werden können. Exemplarisch sei dies an der Überführung der Aminopyridine der Formel IX in die 3-Trifluormethyl-1H-pyrazol-1-yl-substituierten Pyridine der Formel III (mit L¹=Chlor) dargestellt. Der Aufbau des Heterocyclus kann aber auf einer anderen Stufe der dargestellten Varianten A, B und D bis F erfolgen.

Man überführt das Aminopyridin der Formel IX zunächst in die Diazoniumverbindung und erhält nach Hydrierung das entsprechende Pyridinhydrazin-Derivat. Anschließend setzt man mit 1,3-Dicarbonylverbindungen, Enolestern oder 1-Alkinylketonen in einer Cyclokondensation zum gewünschten Pyrazol um:

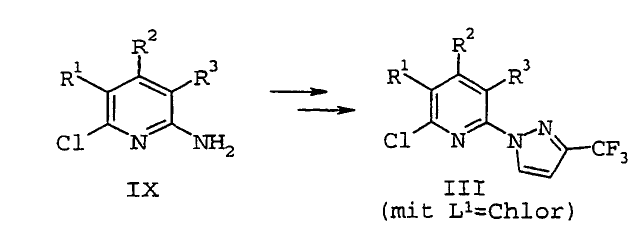

Die erhaltenen 3-Trifluormethyl-1H-pyrazol-1-ylsubstituierten Pyridine der Formel III können dann gemäß der hier vorgestellten Reaktionen weiter modifiziert werden.

Die oben genannten Reaktionen sind im generellen literaturbekannt und werden unter anderem beschrieben in T. Eicher, S. Hauptmann, Chemie der Heterocyclen, 1994, 183; A. S. Tomcufcik, L. N. Starker, The Chemistry of Heterocyclic Compounds, Pyridine and its Derivatives part 3, 1962, 34-35.

### Verfahren D

Bei dieser Variante werden Pyridine der Formel XII zunächst mit einem Pyrazol der Formel IV unter den gleichen Reaktionsbedingungen umgesetzt, unter denen auch die Umsetzung von V zu III (vgl. Verfahren A) erfolgen kann. Anschließend oxidiert man zu Pyridin-N-oxiden der Formel X und durch Halogenierung erhält man 3 -Trifluormethyl-1H-pyrazol-1-yl-substituierte Pyridine der Formel III mit L¹=Hal. Pyrazolylsubstituierte Thienyloxy-Pyridine der Formel I erhält man durch analoge Umsetzung der 3-Trifluormethyl-1H-pyrazol-1-yl-substituierten Pyridine der Formel III mit Hydroxythiophenen der Formel II wie bei Verfahren A beschrieben.

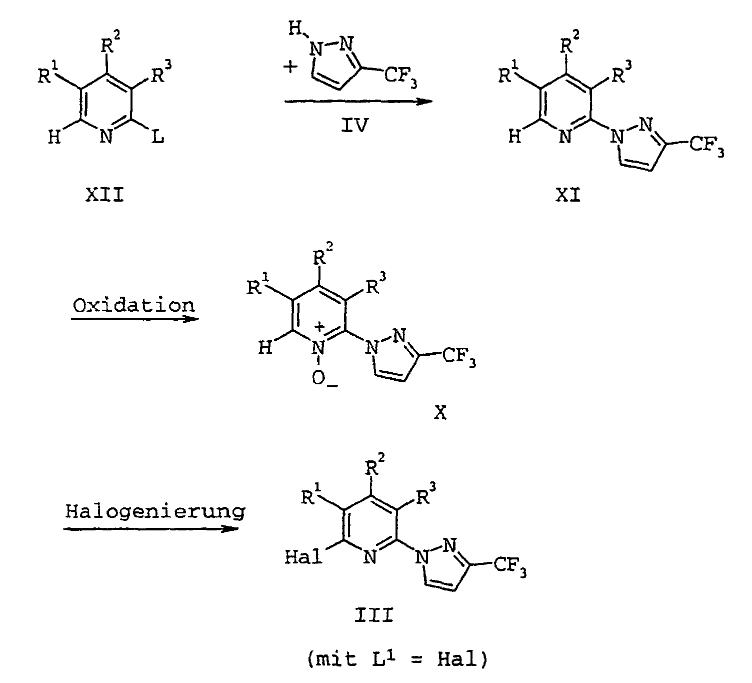

Die Oxidation der Pyridine der Formel XI zu Pyridin-N-oxiden der Formel X erfolgt üblicherweise bei Temperaturen von 0°C bis 100°C, vorzugsweise 0°C bis 25°C, in einem inerten organischen Lösungsmittel [vgl. G. C. Finger et al., J. Am. Chem. Soc. 1959, 81, 2674-2675; M. Tiecco et al., Tetrahedron 1986, 42, 1475-1485].

Geeignete Oxidationsmittel sind z.B. Metachlorperbenzoesäure, Peroxyessigsäure oder Wasserstoffperoxid.

Es kann von Vorteil sein, die Reaktion in Gegenwart eines Katalysators, z.B Natriumwolframat, durchzuführen.

Geeignete Lösungsmittel sind halogenierte Kohlenwasserstoffe wie Methylenchlorid, Chloroform und Chlorbenzol, Alkohole wie Methanol, Ethanol, n-Propanol, Isopropanol, n-Butanol und tert.-Butanol.

Trifluoressigsäure ist ebenfalls ein geeignetes Lösungsmittel.

Die Edukte werden im allgemeinen in äquimolaren Mengen miteinander umgesetzt. Es kann für die Ausbeute vorteilhaft sein, das Oxidationsmittel in einem Überschuß bezogen auf XI einzusetzen.

Die Aufarbeitung kann in an sich bekannter Weise zum Produkt hin erfolgen.

Die Halogenierung der Pyridin-N-oxide der Formel X zu 3-trifluormethyl-1H-pyrazol-1-yl-substituierten Pyridinen der Formel III mit L¹=Hal erfolgt üblicherweise bei Temperaturen von 25°C bis 200°C, vorzugsweise 80°C bis 150°C, in einem inerten organischen Lösungsmittel [vgl. H. E. Mertel, The Chemistry of Heterocyclic Compounds, Pyridine and its Derivatives part 2, 1961, 305-307].

Geeignete Halogenierungsmittel sind z.B. Phosphoroxitrichlorid, Phosphoroxitribromid oder Sulfurylchlorid.

Thionylchlorid ist ebenfalls ein geeignetes Halogenierungsmittel.

Geeignete Lösungsmittel sind aromatische Kohlenwasserstoffe wie Toluol, o-, m- und p-Xylol.

Die Edukte werden im allgemeinen in äquimolaren Mengen miteinander umgesetzt. Es kann für die Ausbeute vorteilhaft sein, das Halogenierungsmittel in einem Überschuß bezogen auf X einzusetzen.

Die Aufarbeitung kann in an sich bekannter Weise zum Produkt hin erfolgen.

### Verfahren E

Thienyloxy-Pyridine der Formel XIII erhält man durch die Umsetzung von Pyridinen der Formel V mit Hydroxythiophenen der Formel II (vgl. EP 955 300). Diese Reaktion erfolgt üblicherweise bei Temperaturen von 25°C bis 200°C, vorzugsweise 80°C bis 150°C analog der Reaktionsbedingungen, wie sie für die Umsetzung von III zu I (vgl. Verfahren A) geschildert wurden. Anschließend werden die Thienyloxy-Pyridine der Formel XIII analog der Umsetzung von V zu III (vgl. Verfahren A) mit Pyrazol-Derivaten der Formel IV zur Reaktion gebracht (vgl. EP 1 101 764):

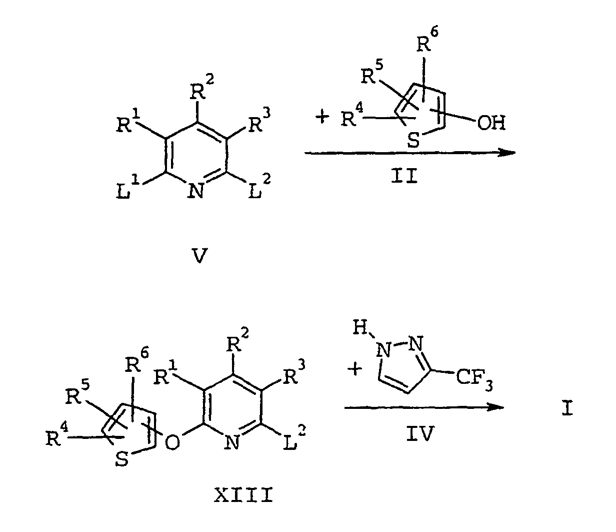

Daneben kann die Umsetzung von XIII zu I auch nickel- oder palladiumkatalysiert erfolgen. Dann erfolgt die Umsetzung üblicherweise bei Temperaturen von 25 °C bis 130 °C in einem inerten organischen Lösungsmittel in Gegenwart einer Base [vgl. B. Gradel et al., Tetrahedron Lett. 2001, 42, 5689-5692; J. F. Hartwig et al., J. Am. Chem. Soc. 1998, 120, 827-828].

L² ist dabei üblicherweise ein Halogenatom wie z.B. Chlor, Brom oder Iod, oder eine andere Abgangsgruppe, wie z.B. Trifluormethylsulfonyloxy.

Als Katalysatoren eignen sich z.B. Nickel- oder Palladiumligandkomplexe, in denen das Metall in der Oxidationsstufe 0 vorliegt und vorzugsweise Nickel- bzw. Palladium(II)salze. Die Umsetzung mit Nickel- bzw. Palladium (II) salzen wird vorzugsweise in Gegenwart von Komplexliganden durchgeführt.

Als Nickel(0)komplexe kommen beispielsweise Nickelcarbenkomplexe in Frage.

Als Palladium(0)komplexligand kommen beispielsweise Tetrakis(triphenylphosphan)palladium, Palladium(diphenylphosphinoferrocen)dichlorid {[PdCl₂(dppf)]} oder Tris(dibenzylidenaceton)dipalladium Pd₂(dba)₃ in Frage.

Als Nickel(II)salze eignen sich beispielsweise Nickelacetat und Nickelacetylacetonat.

Als Palladium(II)salze eignen sich beispielsweise Palladiumacetat und Palladiumchlorid. Bevorzugt wird in Gegenwart von Komplexliganden wie beispielsweise Diphenylphosphinferrocen (dppf) gearbeitet.

Die Herstellung der komplexen Nickelsalze kann in an sich bekannter Weise ausgehend von kommerziell erhältlichen Nickelsalzen wie Nickelchlorid oder Nickelacetat und den entsprechenden Phosphanen wie z.B. Triphenylphosphan oder 1,2-Bis(diphenylphosphano)ethan oder kommerziell erhältlichen Imidazoliniumsalzen erfolgen. Ein Großteil der komplexierten Nickelsalze ist auch kommerziell erhältlich.

Die Herstellung der komplexen Palladiumsalze kann in an sich bekannter Weise ausgehend von kommerziell erhältlichen Palladiumsalzen wie Palladiumchlorid oder Palladiumacetat und den entsprechenden Phosphanen wie z.B. Triphenylphosphan oder 1,2-Bis(diphenylphosphano)ethan erfolgen. Ein Großteil der komplexierten Palladiumsalze ist auch kommerziell erhältlich. Bevorzugte Palladiumsalze sind [(R) (+) 2,2'-Bis(diphenylphosphano)-1,1'-binaphthyl]palladium(II)chlorid, Bis(triphenylphosphan)palladium(II)acetat und insbesondere Bis(triphenylphosphan)palladium(II)chlorid.

Der Katalysator wird in der Regel in einer Konzentration von 0,05 bis 5 Mol%, bevorzugt 1-3 Mol%, eingesetzt.

Geeignete Lösungsmittel sind aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylformamid.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Natriumcarbonat, Kaliumcarbonat und Caesiumcarbonat sowie Alkalimetall- und Erdalkalimetallalkoholate wie Natriummethanolat, Natriumethanolat, Kaliumethanolat und Kalium-tert.-Butylat in Betracht.

Die Basen werden im allgemeinen in äquimolaren Mengen eingesetzt.

Die Edukte werden im allgemeinen in äquimolaren Mengen miteinander umgesetzt. Es kann für die Ausbeute vorteilhaft sein, IV in einem Überschuß bezogen auf XIII einzusetzen.

Die Aufarbeitung kann in an sich bekannter Weise zum Produkt hin erfolgen.

### Verfahren F

Alternativ zu Verfahren E erhält man di-thienyloxysubstituierte Pyridine der Formel XIV durch die Umsetzung von Pyridinen der Formel V mit einem Überschuß des Hydroxythiophens der Formel II (vgl. EP-A-955 300). Bevorzugt wird die Reaktion mit doppeltäquimolarem Verhältnis von II zu V durchgeführt. Diese Reaktion erfolgt analog der Reaktionsbedingungen, wie sie für die Umsetzung von III zu I (vgl. Verfahren A) geschildert wurden. Anschließend werden die di-thienyloxysubstituierten Pyridine der Formel XIV üblicherweise bei Temperaturen von 25°C bis 200°C, vorzugsweise 80°C bis 150°C analog der Umsetzung von V zu III (vgl. Verfahren A) mit Pyrazolen der Formel IV zur Reaktion gebracht (vgl. EP 1 101 764):

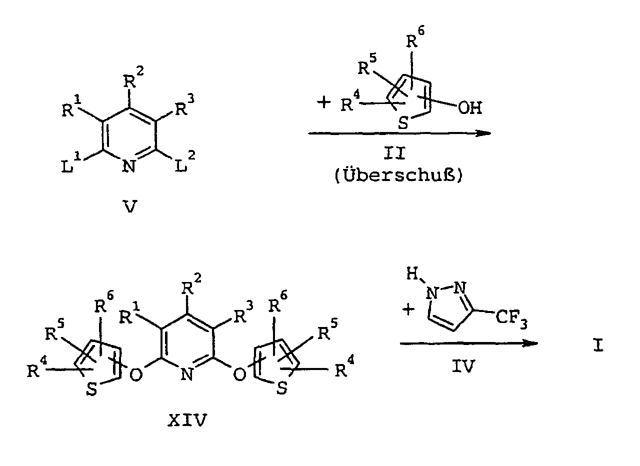

3-Trifluormethyl-1H-pyrazol-1-yl-substituierte Pyridinderivate der Formel III 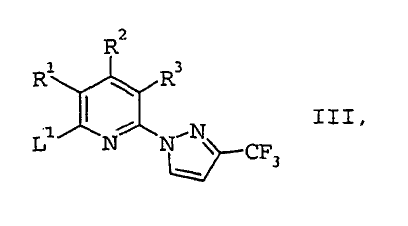 wobei R¹, R² und R³ die für die Verbindungen der Formel I genannten Bedeutungen haben und L¹ für eine nucleophil austauschbare Abgangsgruppe wie Halogen, z.B. Chlor, Brom oder Iod, C₁-C₄-Alkylsulfonyl, C₁-C₄-Alkylsulfonyloxy, C₁-C₄-Halogenalkylsufonyloxy oder Trialkylammonium, bevorzugt sind Fluor, Chlor oder Brom, C₁-C₄-Alkylsulfonyl wie z.B. Methylalkylsulfonyl, oder C₁-C₄-Halogenalkylsufonyloxy, wie z.B. Trifluormethylsulfonyloxy, steht, sind ebenfalls ein Gegenstand der vorliegenden Erfindung.

Die besonders bevorzugten Ausführungsformen der Zwischenprodukte in Bezug auf die Variablen entsprechen denen der Reste R¹, R² und R³ der Formel I.

Besonders bevorzugt sind Verbindungen der Formel III, in denen
- R¹, R³: Wasserstoff, Halogen, Cyano, C₁-C₆-Alkyl oder C₁-C₆-Halogenalkyl;
besonders bevorzugt Wasserstoff, Halogen wie Fluor, Chlor oder Brom, C₁-C₆-Alkyl, wie Methyl oder Ethyl; insbesondere bevorzugt Wasserstoff, Fluor, Chlor oder Methyl; und
- R²: Wasserstoff, Halogen, Cyano, C₁-C₆-Halogenalkyl, C₁-C₆-Alkoxy, C₁-C₆-Alkylthio oder COR⁷;
besonders bevorzugt Wasserstoff, Halogen, wie z.B. Fluor, Chlor oder Brom, Cyano, C₁-C₆-Halogenalkyl wie z.B. Fluormethyl, Chlormethyl, Brommethyl oder Trifluormethyl, C₁-C₆-Alkoxy, wie z.B. Methoxy, oder C₁-C₆-Alkylthio;
insbesondere bevorzugt Wasserstoff, Fluor, Chlor, Cyano, Methoxy oder Trifluormethyl;
bedeuten.

Thienyloxy-Pyridinderivate der Formel XIII 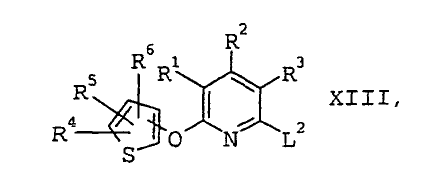 wobei R¹, R², R³, R⁴, R⁵ und R⁶ die für die Verbindungen der Formel I genannten Bedeutungen haben und L² für eine nucleophil austauschbare Abgangsgruppe wie Halogen, z.B. Fluor, Chlor oder Brom, C₁-C₄- Alkylsulfonyl, C₁-C₄-Alkylsulfonyloxy, wie z.B. Methylsulfonyloxy, C₁-C₄-Halogenalkylsufonyloxy oder Trialkylammonium, bevorzugt sind Fluor, Chlor oder Brom, C₁-C₄-Alkylsulfonyl wie z.B. Methylsulfonyl, oder C₁-C₄-Halogenalkylsufonyloxy, wie z.B. Trifluormethylsulfonyloxy, steht, sind ebenfalls Gegenstand der vorliegenden Erfindung.

Die besonders bevorzugten Ausführungsformen der Verbindungen der Formel XIII in Bezug auf die Variablen entsprechen denen der Reste R¹, R², R³, R⁴, R⁵ und R⁶ der Formel I.

Besonders bevorzugt werden die Verbindungen der Formel XIII, in denen L² für Halogen, wie z.B. Fluor oder Chlor, steht.

Bevorzugt werden Verbindungen der Formel XIII, in denen
- R¹, R³: Wasserstoff, Halogen, Cyano, C₁-C₆-Alkyl oder C₁-C₆-Halogenalkyl ;
besonders bevorzugt Wasserstoff, Halogen wie Fluor, Chlor oder Brom, C₁-C₆-Alkyl, wie Methyl oder Ethyl;
insbesondere bevorzugt Wasserstoff, Fluor, Chlor oder Methyl
- R²: Wasserstoff, Halogen, Cyano, C₁-C₆-Halogenalkyl, C₁-C₆-Alkoxy, C₁-C₆-Alkylthio oder COR⁷;
besonders bevorzugt Wasserstoff, Halogen, wie z.B. Fluor, Chlor oder Brom, Cyano oder C₁-C₆-Halogenalkyl wie z.B. Fluormethyl, Chlormethyl, Brommethyl oder Trifluormethyl, C₁-C₆-Alkoxy, wie z.B. Methoxy, oder C₁-C₆-Alkylthio;
insbesondere bevorzugt Wasserstoff, Fluor, Chlor, Cyano, Methoxy oder Trifluormethyl; und
- R⁴, R⁵, R⁶: Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Alkylsulfonyl oder C₁-C₆-Halogenalkylsufonyl;
besonders bevorzugt Wasserstoff, Halogen, Cyano, C₁-C₆-Alkyl, C₁-C₆-Halogenalkyl, C₁-C₆-Halogenalkoxy, C₁-C₆-Alkylsulfonyl oder C₁-C₆-Halogenalkylsufonyl;
insbesondere bevorzugt Wasserstoff, Halogen, C₁-C₆-Halogenalkyl oder C₁-C₆-Halogenalkoxy;
sehr bevorzugt Wasserstoff, Fluor, Chlor, Trifluormethyl oder Difluormethoxy;
bedeuten.

### Herstellungsbeispiele:

### Gemäß Verfahren E:

### 2,3,5-Trifluor-6-(5-trifluormethyl-3-thienyloxy)pyridin

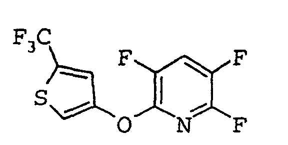

3 g (19.9 mmol) 2,3,5,6-Tetrafluorpyridin, 3.34 g (19.9 mmol) 5-Trifluormethyl-3-hydroxythiophen und 5.48 g (39.7 mmol) Kaliumcarbonat wurden in 30 ml DMF 12 h bei Raumtemperatur gerührt. Nach Verdünnen mit 200 ml Wasser wurde mit Diethylether extrahiert. Die organische Phase wurde gewaschen, getrocknet und vom Lösungsmittel befreit. Man erhielt 4.86 g (16.3 mmol, 82%) der Titelverbindung.
¹H-NMR (400 MHz, CDCl₃): δ = 7.3 (s, 1H), 7.35 (s, 1H), 7.5 (m, 1H)

### 3,5-Difluor-2-(3-trifluormethyl-1H-pyrazol-1-yl)-6-(5-trifluormethyl-3-thienyloxy)pyridin

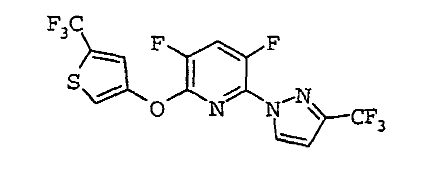

Eine Mischung von 0.2 g (0.67 mmol) 2,3,5-Trifluor-6-(5-trifluormethyl-3-thienyloxy)pyridin, 0.08 g (0.59 mmol) 3-Trifluormethyl-1H-pyrazol und 0.14 g (0.1 mmol) Kaliumcarbonat wurde in 20 ml N,N-Dimethylformamid (DMF) 12 h auf 80°C erhitzt. Anschließend wurde mit Wasser und Essigsäureethylester verdünnt. Die wäßrige Phase wurde mit Essigsäureethylester extrahiert, die vereinigten organischen Phasen wurden gewaschen, getrocknet und vom Lösungsmittel befreit. Nach Säulenchromatographie (Petrolether/MTBE 8:1 → 3:1) erhielt man 0.15 g (0.36 mmol, 61%) der Titelverbindung.
¹H-NMR (400 MHz, CDCl₃): δ = 6.7 (s, 1H), 7.3 (s, 1H), 7.4 (s, 1H) , 7.6 (t, 1H) , 8.0 (s, 1H)

### Gemäß Verfahren A:

### 2-Chlor-4-methoxy-6- (3-trifluormethyl-1H-pyrazolyl)pyridin

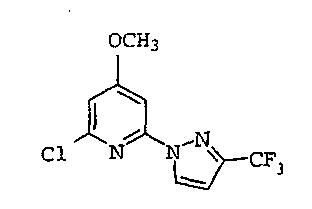

Eine Mischung aus 1 g (5.6 mmol) 2,6-Dichlor-4-methoxypyridin, 0.72 g (5.3 mmol) 3-Trifluormethylpyrazol, 3.7 g (11 mmol)Cäsiumcarbonat, 2 Spatelspitzen Bis[Kupfer(I)-trifluormethansulfonat] benzolkomplex und 3 Tropfen Essigsäureethylester in Xylol wurde 23 h bei 120 °C gerührt. Nach Verdünnen mit Wasser wurde mit Essigsäureethylester extrahiert. Die vereinigten organischen Phasen wurden gewaschen, getrocknet und vom Lösungsmittel befreit. Man erhielt nach Säulenchromatographie (Petrolether/Essigsäureethylester 100:0 -> 0:100) 0.9 g (3.2 mmol, 61 %) der Titelverbindung.

### 4-Methoxy-6-(3-trifluormethyl-1H-pyrazol-1-yl)-2-(5-trifluormethyl-3-thienyloxy)pyridin

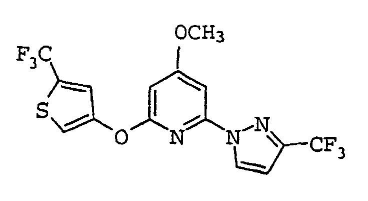

Eine Mischung aus 182 mg (1.1 mmol) 3-Hydroxy-5-trifluormethylthiophen, 299 mg (2.2 mmol) Kaliumcarbonat, 1 Spatelspitze 18-Krone-6 und 300 mg (1.1 mmol) 2-Chlor-4-methoxy-6-(3-trifluormethyl-1H-pyrazolyl)pyridin wurde 5.5 h bei 120 °C in 8 ml N-Methylpyrrolidon (NMP) gerührt. Der Ansatz wurde mit MTBE/Wasser 1:1 versetzt und mit Methyl-tert.-butylether (MTBE) extrahiert. Die vereinigten organischen Phasen wurden gewaschen, getrocknet und vom Lösungsmittel befreit. Nach Chromatographie erhielt man 200 mg (0.5 mmol, 44 %) der Titelverbindung.

### Gemäß Verfahren B:

### 2-Chlor-4-cyano-6-methylthiopyridin

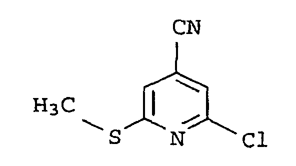

1.04 g (6 mmol) 2,6-Dichlor-4-cyanopyridin wurden mit 0.42 g (6 mmol) Natriumthiomethylat in THF 13 h unter Rückfluss gerührt. Nach Entfernen des Lösungsmittels im Vakuum wurde der Rückstand in Wasser aufgenommen und mit Essigsäureethylester extrahiert. Die vereinigten organischen Phasen wurden getrocknet, eingeengt und durch Säulenchromatographie (Cyclohexan/Essigester 7:1 -> 2:1) gereinigt, wodurch man 0.67 g (3.64 mmol, 61 %) der Titelverbindung erhielt.

### 4-Cyano-2-methylthio-6-(3-trifluormethyl-1H-pyrazol-1-yl)pyridin

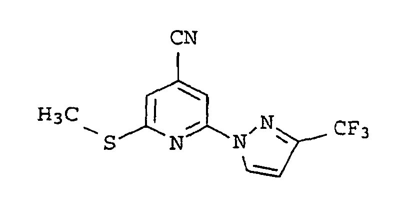

Eine Mischung aus 0.65 g (3.52 mmol) 2-Chlor-4-cyano-6-methylthiopyridin, 0.43 g (3.17 mmol) 3-Trifluormethylpyrazol und 0.75 g (5.28 mmol) Kaliumcarbonat in DMF wurde 7 h bei 50 °C und 72 h bei Raumtemperatur gerührt. Das Reaktionsgemisch wurde mit Wasser verdünnt und mit Essigsäureethylester extrahiert. Die vereinigten organischen Phasen wurden getrocknet und vom Lösungsmittel befreit, worauf man 0.59 g (2,08 mmol, 55 %) der Titelverbindung erhielt.

### 4-Cyano-2-methylsulfonyl-6-(3-trifluormethyl-1H-pyrazol-1-yl) pyridin

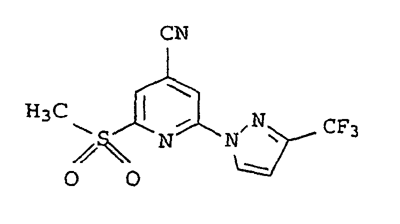

Zu 560 mg (1.97 mmol) 2-Cyano-2-methylthio-6-(3-trifluormethyl-1H-pyrazol-1-yl)pyridin in Methanol wurden bei 0-5 °C 1.82 g (2.96 mmol) Oxon® in Wasser getropft. Der pH-Wert der Lösung wurde bei 2-3 gehalten. Das Reaktionsgemisch wurde 14 h bei Raumtemperatur gerührt, mit Wasser verdünnt und mit Essigsäureethylester extrahiert. Die vereinigten organischen Phasen wurden gewaschen, getrocknet und vom Lösungsmittel befreit. Man erhielt 540 mg (1.71 mmol, 87 %) der Titelverbindung.

### 4-Cyano-6-(3-trifluormethyl-1H-pyrazol-1-yl)-2-(5-trifluormethyl-3-thienyloxy)pyridin

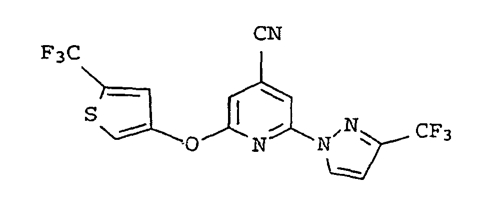

Eine Mischung aus 270 mg (1.62 mmol) 3-Hydroxy-5-trifluormethylthiophen, 540 mg (1.71 mmol) 4-Cyano-2-methylsulfonyl-6-(3-trifluormethyl-1H-pyrazol-1-yl)pyridin und 350 mg (2.57 mmol) Kaliumcarbonat wurde in DMF 7 h bei 80 °C und 72 h bei Raumtemperatur gerührt. Der Ansatz wurde eingeengt, mit Wasser versetzt und mit Essigsäureethylester extrahiert. Anschließend wurden die vereinigten organischen Phasen getrocknet und vom Lösungsmittel befreit. Nach Säulenchromatographie (Petrolether/Essigsäureethylester 100:0 -> 0:100) erhielt man 500 mg (1.24 mmol, 76 %) der Titelverbindung.

### Gemäß Verfahren D:

### 5-Methyl-2- (3-trifluormethyl-1H-pyrazol-1-yl)pyridin

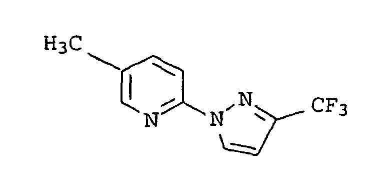

Eine Mischung aus 3.03 g (17.6 mmol) 2-Brom-5-methylpyridin, 3,59 g (26.4 mmol) 3-Trifluormethylpyrazol, 6.3 g (19.3 mmol) Cäsiumcarbonat, 3.17 g (17.6 mmol) Phenanthrolin, 2.06 g (8.8 mmol) Dibenzylidenaceton und einer Spatelspitze Bis [Kupfer(I)-trifluormethansulfonat]benzolkomplex in Xylol wurde 8 h bei 125 °C und 12 h bei Raumtemperatur gerührt. Das Reaktionsgemisch wurde mit Diethylether verdünnt und die organische Phase mit gesättigter Ammoniumchlorid- und mit gesättigter Natriumchloridlösung gewaschen. Nach Trocknen und Entfernen des Lösungsmittels wurde das Reaktionsgemisch durch Säulenchromatographie (Petrolether/MTBE 100:0 -> 50:50) gereinigt, wodurch man 3.0 g (13.2 mmol, 75 %) der Titelverbindung erhielt.

### 5-Methyl-2-(3-trifluormethyl-1H-pyrazol-1-yl)pyridin-N-oxid

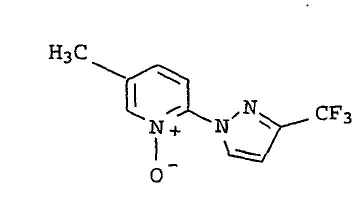

Zu 2.6 g (11 mmol) 5-Methyl-2-(3-trifluormethyl-1H-pyrazol-1-yl) pyridin in Trifluoressigsäure wurden 2 Spatelspitzen Natriumwolframat und insgesamt 11.9 ml 30 %ige Wasserstoffperoxidlösung gegeben. Nach 96 h bei Raumtemperatur wurde das Reaktionsgemisch mit Wasser verdünnt und mit Essigsäureethylester extrahiert. Anschließend wurden die vereinigten organischen Phasen gewaschen, getrocknet und vom Lösungsmittel befreit. Nach Säulenchromatographie (Cyclohexan/Essigsäureethylester 95:5 -> 0:100) erhielt man 1.6 g (6.6 mmol, 60 %) der Titelverbindung.

### 2-Chlor-3-methyl-6-(3-trifluormethyl-1H-pyrazol-1-yl)pyridin

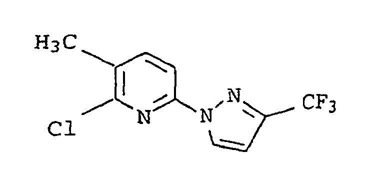

Zu 2.15 g (14 mmol) Phosphoroxytrichlorid wurden bei 80 °C portionsweise 1.7 g (7 mmol) 5-Methyl-2- (3-trifluormethyl-1H-pyrazol-1-yl)pyridin-N-oxid gegeben und 4 h bei dieser Temperatur gerührt. Unter Kühlung wurde das Reaktionsgemisch hydrolysiert und mit Essigsäureethylester extrahiert. Anschließend wurden die vereinigten organischen Phasen gewaschen, getrocknet und vom Lösungsmittel befreit. Nach Säulenchromatographie (Cyclohexan/Essigsäureethylester 100:9 -> 95:5) erhielt man 1.1 g (4.2 mmol, 60 %) der Titelverbindung.

### 3-Methyl-6-(3-trifluormethyl-1H-pyrazol-1-yl)-2-(5-trifluormethyl-3-thienyloxy)pyridin

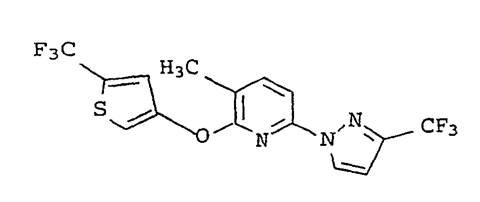

Eine Mischung aus 0.38 g (2.3 mmol) 3-Hydroxy-5-trifluormethylthiophen, 0.5 g (1.9 mmol 2-Chlor-3-methyl-6-(3-trifluormethyl-1H-pyrazol-1-yl)pyridin, 0.14 g (1 mmol) Kupfer(I)bromid und 0.52 g (3.8 mmol) Kaliumcarbonat wurde in DMF 15 h bei 120 °C und 60 h bei Raumtemperatur gerührt. Der Ansatz wurde eingeengt, mit Wasser versetzt und mit Essigsäureethylester extrahiert. Anschließend wurden die vereinigten organischen Phasen gewaschen, getrocknet und vom Lösungsmittel befreit. Nach Chromatographie (Kieselgel RP-18, Methanol/Wasser 8:2 -> 9:1) erhielt man 0.15 g (0.4 mmol, 20 %) der Titelverbindung.

In den Tabellen 2 und 3 sind neben den voranstehenden Verbindungen noch weitere pyrazolylsubstituierte Thienyloxypyridine der Formel I sowie Thienyloxy-Pyridine der Formel XIII aufgeführt,
die in analoger Weise nach den voranstehend beschriebenen Verfahren hergestellt wurden oder herstellbar sind.

In Tabelle 4 werden neben den voranstehenden Verbindungen noch weitere 3-Trifluormethyl-1H-pyrazol-1-yl-substituierte Pyridine der Formel III genannt, die in analoger Weise nach den voranstehend beschriebenen Verfahren hergestellt wurden oder herstellbar sind.

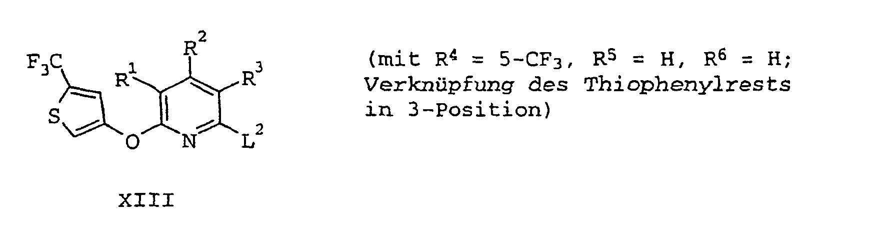

**Tabelle 2**

| Nr. | R¹ | R² | R³ | L² | ¹H-NMR [400 MHz, CDCl₃] |
|---|---|---|---|---|---|
| 2.1 | F | H | F | F | 7.3 (s, 1H), 7.35 (s, 1H), 7.5 (m, 1H) |

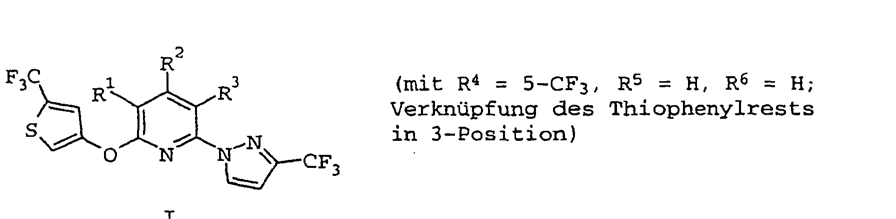

**Tabelle 3**

| Nr. | R¹ | R² | R³ | ¹H-NMR (400 MHz, CDCl₃) |
|---|---|---|---|---|
| 3.1 | H | H | H | 6.7 (s, 1H), 6.9 (d, 1H), 7.2 (s, 1H), 7.4 (s, 1H), 7.8 (d, 1H), 7.9 (t, 1H), 8.2 (s, 1H) |
| 3.2 | H | CN | H | 6.7 (s, 1H), 7.1 (s, 1H), 7.3 (s, 1H), 8.0 (s, 1H), 8.2 (s, 1H) |
| 3.3 | H | OCH₃ | H | 4.0 (s, 1H), 6.3 (s, 1H), 6.6 (s, 1H), 7.2 (s, 1H), 7.3 (s, 1H), 7.4 (s, 1H), 8.2 (s, 1H) |
| 3.4 | CN | H | H | 6.7 (s, 1H), 7.3 (s, 1H), 7.4 (s, 1H), 7.8 (d, 1H), 8.1 (s, 1H), 8.2 (d, 1H) |
| 3.5 | CF₃ | H | H | 6.7 (s, 1H), 7.3 (s, 1H), 7.4 (s, 1H), 7.8 (d, 1H), 8.1 (s, 1H); 8.2 (d, 1H) |
| 3.6 | F | H | F | 6.7 (s, 1H), 7.3 (s, 1H), 7.4 (s, 2H) , 7.6 (t, 1H) , 8.0 (s, 1H) |
| 3.7 | Cl | Cl | Cl | 6.8 (s, 1H), 7.1 (s, 1H), 7.2 (s, 1H), 7.7 (s, 1H) |
| 3.8 | CH₃ | H | H | 2.4 (s, 3H), 6.6 (d, 1H), 7.2 (s, 1H) , 7.4 (s, 1H) , 7.6 (d, 1H), 7.7 (d, 1H), 8.1 (d, 1H) |

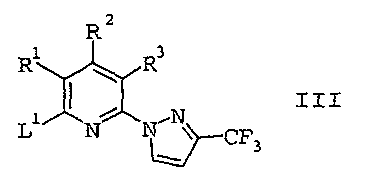

**Tabelle 4**

| Nr. | R¹ | R² | R³ | L¹ | ¹H-NMR (400 MHZ, CDCl₃ |
|---|---|---|---|---|---|
| 4.1 | H | OCH₃ | H | Cl | 4.0 (s, 3H), 6.7 (s, 1H), 6.8 (s, 1H), 7.5 (s, 1H), 8.6 (s, 1H) |
| 4.2 | H | CN | H | SO₂CH₃ | 3.3 (s, 3H), 6.8 (s, 1H) 8.2 (s, 1H), 8.5 (s, 1H), 8.6 (s, 1H) |
| 4.3 | CH₃ | H | H | Cl | 6.7 (s, 1H), 7.7 (d, 1H), 7.9 (d, 1H), 8.6 (s, 1H) |
| 4.4 | H | H | H | SO₂CH₃ | 3.2 (s, 3H), 6.8 (s, 1H), 8.0 (d, 1H), 8.1 (t. 1H), 8.3 (d, 1H), 8.6 (s, 1H) |
| 4.5 | CN | H | H | Cl | 6.8 (s, 1H), 8.1 (d, 1H), 8.1 (d, 1H), 8.6 (s, 1H) |
| 4.6 | CF₃ | H | H | Cl | 6.7 (s, 1H), 8.1 (d, 1H), 8.2 (d, 1H), 8.6 (s, 1H) |
| 4.7 | Cl | Cl | Cl | Cl | 6.5 (s, 1H), 7.2 (s, 1H) |

### Anwendung

Die pyrazolylsubstituierten Thienyloxy-Pyridine der Formel I und deren landwirtschaftlich brauchbaren Salze eignen sich - sowohl als Isomerengemische als auch in Form der reinen Isomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:

Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile eines Wirkstoffs der Formel I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile eines Wirkstoffs der Formel I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile eines Wirkstoffs der Formel I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile eines Wirkstoffs der Formel I werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile eines Wirkstoffs der Formel I werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile eines Wirkstoffs der Formel I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil eines Wirkstoffs der Formel I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil eines Wirkstoffs der Formel I wird in einer Mischung gelöst, die aus 80 Gewichtsteilen Cyclohexanon und 20 Gewichtsteilen Wettol^{R} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.).

Zur Verbreiterung des Wirkungsspektrums und zur Erzielung synergistischer Effekte können die pyrazolylsubstituierten Thienyloxy-Pyridine der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone,. Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. Pyrazolylsubstituierte Thienyloxy-Pyridine der Formel I 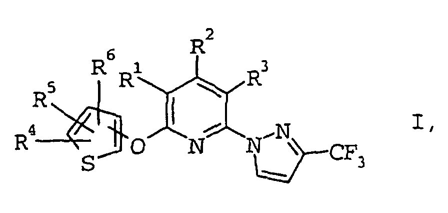 in der die Variablen die folgenden Bedeutungen haben:
R¹, R³ Wasserstoff, Halogen, Cyano, Nitro, C₁-C₆-Alkyl, C₁-C₆-Halogenalkyl, C₁-C₆-Alkoxy oder C₁-C₆-Halogenalkoxy;
R² Wasserstoff, Halogen, Cyano, C₂-C₆-Alkenyl, C₂-C₆-Alkinyl, C₁-C₆-Halogenalkyl, C₂-C₆-Halogenalkenyl, C₂-C₆-Halogenalkinyl, C₁-C₆-Alkoxy, C₃-C₆-Alkenyloxy, C₃-C₆-Alkinyloxy, C₁-C₆-Halogenalkoxy, C₁-C₆-Alkoxy-C₁-C₄-alkyl, C₁-C₆-Alkylamino, Di-(C₁-C₄-alkyl)amino, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl oder COR⁷;
R⁴, R⁵, R⁶ Wasserstoff, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfonyl oder C₁-C₆-Halogenalkylsulfonyl;
R⁷ Wasserstoff, Hydroxy, C₁-C₆-Alkyl, C₁-C₆-Alkoxy, Amino, C₁-C₆-Alkylamino oder Di-(C₁-C₄-alkyl)amino;
sowie deren landwirtschaftlich brauchbaren Salze.

2. Pyrazolylsubstituierte Thienyloxy-Pyridine der Formel I nach Anspruch 1, in der die Variablen
R¹, R³ Wasserstoff, Halogen, Cyano, Nitro, C₁-C₆-Alkyl oder C₁-C₆-Halogenalkyl;
R² Wasserstoff, Halogen, Cyano, C₁-C₆-Halogenalkyl, C₁-C₆-Alkoxy, C₁-C₆-Alkylamino, Di(C₁-C₄-alkyl)amino, C₁-C₆-Alkylthio oder COR⁷
bedeuten.

3. Pyrazolylsubstituierte Thienyloxy-Pyridine der Formel I nach Anspruch 1 oder 2, in der die Variablen
R⁴, R⁵, R⁶ Wasserstoff, Halogen, Cyano, C₁-C₆-Alkyl, C₁-C₆-Halogenalkyl oder C₁-C₆-Halogenalkoxy;
bedeuten.

4. Verfahren zur Herstellung von pyrazolylsubstituierten Thienyloxy-Pyridinen der Formel I gemäß Anspruch 1, **dadurch gekennzeichnet, daß** man 3-Trifluormethyl-1H-pyrazol-1-yl-substituierte Pyridine der Formel III 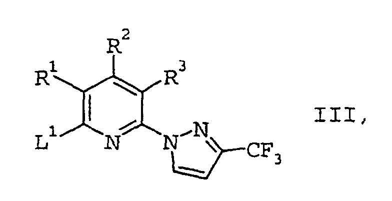 wobei R¹, R² und R³ die unter Anspruch 1 genannten Bedeutungen haben und L¹ für eine nucleophil austauschbare Abgangsgruppe steht,
mit einem Hydroxythiophen der Formel II 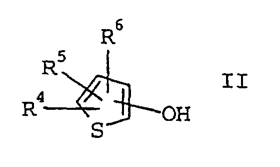 wobei R⁴, R⁵ und R⁶ die unter Anspruch 1 genannten Bedeutungen haben, umsetzt.

5. Verfahren zur Herstellung von pyrazolylsubstituierten Thienyloxy-Pyridinen der Formel I gemäß Anspruch 1, **dadurch gekennzeichnet, daß** man Thienyloxy-Pyridinderivate der Formel XIII 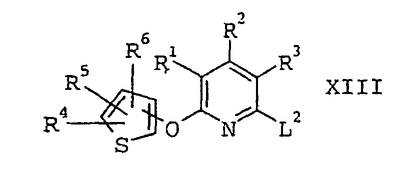 wobei R¹, R², R³, R⁴, R⁵ und R⁶ die unter Anspruch 1 genannten Bedeutungen haben und L² für eine nucleophil austauschbare Abgangsgruppe steht,
mit einem Pyrazol-Derivat der Formel IV 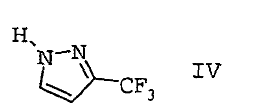 umsetzt.

6. 3-Trifluormethyl-1H-pyrazol-1-yl-substituierte Pyridine der Formel III 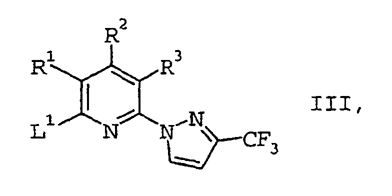 wobei R¹, R² und R³ die unter Anspruch 1 genannten Bedeutungen haben und L¹ für eine nucleophil austauschbare Abgangsgruppe steht.

7. Thienyloxy-Pyridinderivate der Formel XIII 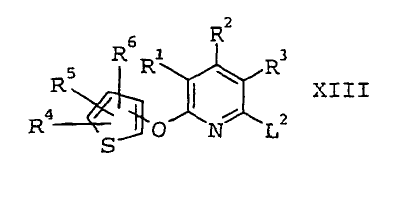 wobei R¹, R², R³, R⁴, R⁵ und R⁶ die unter Anspruch 1 genannten Bedeutungen haben und L² für eine nucleophil austauschbare Abgangsgruppe steht.

8. Mittel, enthaltend eine herbizid wirksame Menge mindestens eines pyrazolylsubstituierten Thienyloxy-Pyridins der Formel I oder eines landwirtschaftlich brauchbaren Salzes von I gemäß den Ansprüchen 1 bis 3 und für die Formulierung von Pflanzenschutzmitteln übliche Hilfsmittel.

9. Verfahren zur Herstellung von Mitteln gemäß Anspruch 8, **dadurch gekennzeichnet, daß** man eine herbizid wirksame Menge mindestens eines pyrazolylsubstituierten Thienyloxy-Pyridinderivates der Formel I oder eines landwirtschaftlich brauchbaren Salzes von I gemäß den Ansprüchen 1 bis 3 und für die Formulierung von Pflanzenschutzmitteln übliche Hilfsmittel mischt.

10. Verfahren zur Bekämpfung von unerwünschtem Pflanzenwuchs, **dadurch gekennzeichnet, daß** man eine herbizid wirksame Menge mindestens eines pyrazolylylsubstituierten Thienyloxy-Pyridinderivates der Formel I oder eines landwirtschaftlich brauchbaren Salzes von I gemäß den Ansprüchen 1 bis 3 auf Pflanzen, deren Lebensraum und/oder auf Samen einwirken läßt.

11. Verwendung der pyrazolylsubstituierten Thienyloxy-Pyridinderivate der Formel I und deren landwirtschaftlich brauchbaren Salze gemäß den Ansprüchen 1 bis 3 als Herbizide.

## Claims

1. A pyrazolyl-substituted thienyloxypyridine of the formula I 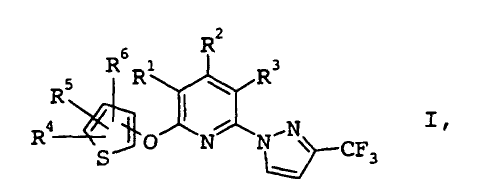 where
R¹, R³ are hydrogen, halogen, cyano, nitro, C₁-C₆-alkyl, C₁-C₆-haloalkyl, C₁-C₆-alkoxy or C₁-C₆-haloalkoxy;
R² is hydrogen, halogen, cyano, C₂-C₆-alkenyl, C₂-C₆-alkynyl, C₁-C₆-haloalkyl, C₂-C₆-haloalkenyl, C₂-C₆-haloalkynyl, C₁-C₆-alkoxy, C₃-C₆-alkenyloxy, C₃-C₆-alkynyloxy, C₁-C₆-haloalkoxy, C₁-C₆-alkoxy-C₁-C₄-alkyl, C₁-C₆-alkylamino, di(C₁-C₄-alkyl)amino, C₁-C₆-alkylthio, C₁-C₆-haloalkylthio, C₁-C₆-alkylsulfinyl, C₁-C₆-haloalkylsulfinyl, C₁-C₆-alkylsulfonyl, C₁-C₆-haloalkylsulfonyl or COR⁷;
R⁴, R⁵, R⁶ are hydrogen, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfonyl or C₁-C₆-haloalkylsulfonyl;
R⁷ is hydrogen, hydroxyl, C₁-C₆-alkyl, C₁-C₆-alkoxy, amino, C₁-C₆-alkylamino or di(C₁-C₄-alkyl)amino;
and its agriculturally useful salts.

2. A pyrazolyl-substituted thienyloxypyridine of the formula I as claimed in claim 1 where
R¹, R³ are hydrogen, halogen, cyano, nitro, C₁-C₆-alkyl or C₁-C₆-haloalkyl;
R² is hydrogen, halogen, cyano, C₁-C₆-haloalkyl, C₁-C₆-alkoxy, C₁-C₆-alkylamino, di(C₁-C₄-alkyl)amino, C₁-C₆-alkylthio or COR⁷.

3. A pyrazolyl-substituted thienyloxypyridine of the formula I as claimed in claim 1 or 2 where
R⁴, R⁵, R⁶ are hydrogen, halogen, cyano, C₁-C₆-alkyl, C₁-C₆-haloalkyl or C₁-C₆-haloalkoxy.

4. A process for preparing a pyrazolyl-substituted thienyloxypyridine of the formula I as claimed in claim 1, which comprises reacting 3-trifluoromethyl-1H-pyrazol-1-yl-substituted pyridines of the formula III 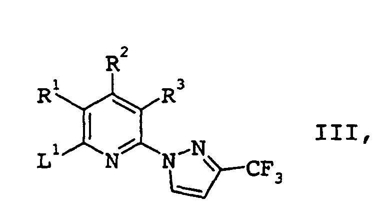 where R¹, R² and R³ are as defined in claim 1 and L¹ is a nucleophilically displaceable leaving group,
with a hydroxythiophene of the formula II 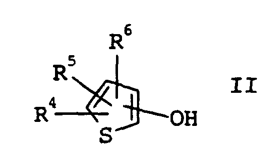 where R⁴, R⁵ and R⁶ are as defined in claim 1.

5. A process for preparing pyrazolyl-substituted thienyloxypyridines of the formula I as claimed in claim 1, which comprises reacting thienyloxypyridine derivatives of the formula XIII 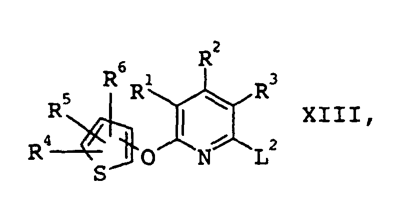 where R¹, R², R³, R⁴, R⁵ and R⁶ are as defined in claim 1 and L² is a nucleophilically displaceable leaving group,
with a pyrazole derivative of the formula IV

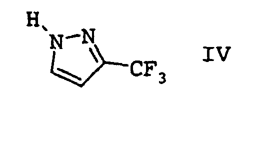

6. A 3-trifluoromethyl-1H-pyrazol-1-yl-substituted pyridine of the formula III 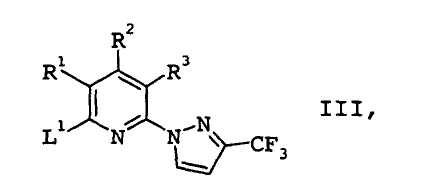 where R¹, R² and R³ are as defined in claim 1 and L¹ is a nucleophilically displaceable leaving group.

7. A thienyloxypyridine derivative of the formula XIII 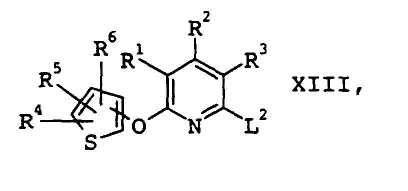 where R¹, R², R³, R⁴, R⁵ and R⁶ are as defined in claim 1 and L² is a nucleophilically displaceable leaving group.

8. A composition, comprising a herbicidally effective amount of at least one pyrazolyl-substituted thienyloxypyridine of the formula I or of an agriculturally useful salt of I as claimed in any of claims 1 to 3 and auxiliaries customary for formulating crop protection agents.

9. A process for preparing compositions as claimed in claim 8, which comprises mixing a herbicidally effective amount of at least one pyrazolyl-substituted thienyloxypyridine derivative of the formula I or of an agriculturally useful salt of I as claimed in any of claims 1 to 3 and auxiliaries customary for formulating crop protection agents.

10. A method for controlling undesirable vegetation, which comprises allowing a herbicidally effective amount of at least one pyrazolyl-substituted thienyloxypyridine derivative of the formula I or of an agriculturally useful salt of I as claimed in any of claims 1 to 3 to act on plants, their habitat and/or on seeds.

11. The use of the pyrazolyl-substituted thienyloxypyridine derivatives of the formula I and their agriculturally useful salts as claimed in any of claims 1 to 3 as herbicides.

## Revendications

1. Thiényloxy-pyridines substituées par du pyrazolyle de la formule I : 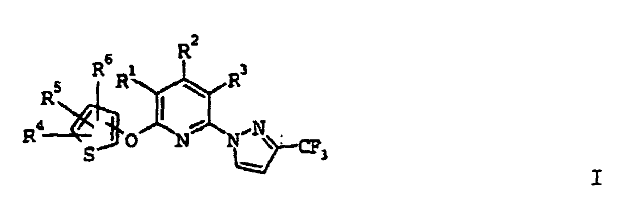 dans laquelle les variables ont les significations suivantes :
R¹, R³ représentent de l'hydrogène, de l'halogène ou un groupe cyano, nitro, alkyle en C₁-C₆, halogénoalkyle en C₁-C₆, alcoxy en C₁-C₆ ou halogénoalcoxy en C₁-C₆,
R² représente de l'hydrogène, de l'halogène ou un groupe cyano, alcényle en C₂-C₆, alcynyle en C₂-C₆, halogénoalkyle en C₁-C₆, halogénoalcényle en C₂-C₆, halogénoalcynyle en C₂-C₆, alcoxy en C₁-C₆, alcényloxy en C₃-C₆, alcynyloxy en C₃-C₆, halogénoalcoxy en C₁-C₆, (alcoxy en C₁-C₆)-alkyle en C₁-C₄-, alkylamino en C₁-C₆, di-(alkyle en C₁-C₄)-amino, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆, halogénoalkylsulfonyle en C₁-C₆, ou COR⁷,
R⁴, R⁵, R⁶ représentent de l'hydrogène, de l'halogène ou un groupe cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆, alkylsulfonyle en C₁-C₆ ou halogénoalkylsulfonyle en C₁-C₆,
R⁷ représente de l'hydrogène ou un groupe hydroxy, alkyle en C₁-C₆, alcoxy en C₁-C₆, amino, alkylamino en C₁-C₆ ou di-(alkyle en C₁-C₄)-amino,
ainsi que leurs sels utilisables en agriculture.

2. Thiényloxy-pyridines substituées par du pyrazolyle de la formule I suivant la revendication 1, dans laquelle les variables
R¹, R³ représentent de l'hydrogène, de l'halogène ou un groupe cyano, nitro, alkyle en C₁-C₆ ou halogénoalkyle en C₁-C₆,
R² représente de l'hydrogène, de l'halogène ou un groupe cyano, halogénoalkyle en C₁-C₆, alcoxy en C₁-C₆, alkylamino en C₁-C₆, di-(alkyle en C₁-C₄)-amino, alkylthio en C₁-C₆ ou COR⁷.

3. Thiényloxy-pyridines substituées par du pyrazolyle de la formule I suivant l'une des revendications 1 et 2, dans laquelle les variables
R⁴, R⁵, R⁶ représentent de l'hydrogène, de l'halogène ou un groupe cyano, alkyle en C₁-C₆, halogénoalkyle en C₁-C₆ ou halogénoalcoxy en C₁-C₆,

4. Procédé de préparation de thiényloxy-pyridines substituées par du pyrazolyle de la formule I suivant la revendication 1, **caractérisé en ce qu'**on fait réagir des pyridines substituées par du 3-trifluorométhyl-1H-pyrazol-1-yle de la formule III : 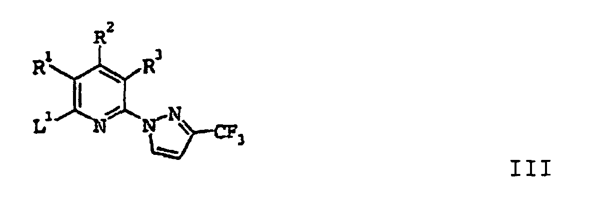 dans laquelle R¹, R² et R³ ont les significations données dans la revendication 1 et L¹ représente un groupe sortant échangeable par voie nucléophile, avec de l'hydroxythiophène de la formule II : 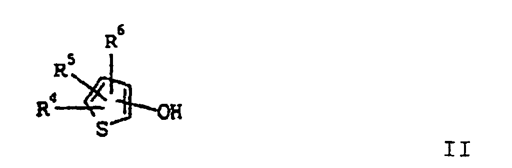 dans laquelle R⁴, R⁵ et R⁶ ont les significations données dans la revendication 1.

5. Procédé de préparation de thiényloxy-pyridines substituées par du pyrazolyle de la formule I suivant la revendication 1, **caractérisé en ce qu'**on fait réagir des dérivés de thiényloxy-pyridines de la formule XIII : 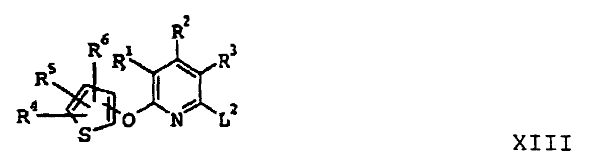 dans laquelle R¹, R², R³, R⁴, R⁵ et R⁶ ont les significations données dans la revendication 1 et L² représente un groupe sortant échangeable par voie nucléophile,
avec un dérivé de pyrazole de la formule IV :

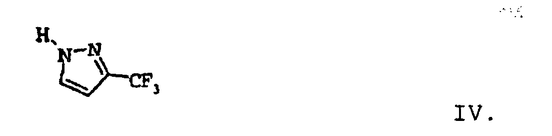

6. Pyridines substituées par du 3-trifluorométhyl-1H-pyrazol-1-yle de la formule III : 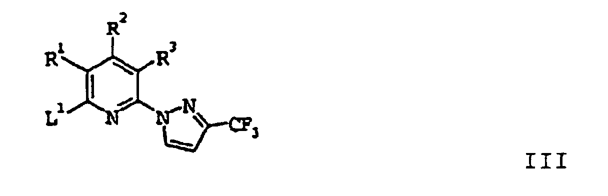 dans laquelle R¹, R² et R³ ont les significations données dans la revendication 1 et L¹ est un groupe sortant échangeable par voie nucléophile.

7. Dérivés de thiényloxy-pyridine de la formule XIII : 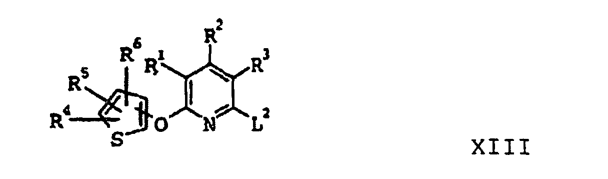 dans laquelle R¹, R², R³, R⁴, R⁵ et R⁶ ont les significations données dans la revendication 1 et L² est un groupe sortant échangeable par voie nucléophile.

8. Produits, contenant une quantité efficace du point de vue herbicide d'au moins une thiényloxy-pyridine substituée par du pyrazolyle de la formule I ou d'un sel utilisable en agriculture de I suivant l'une des revendications 1 à 3 et des adjuvants courants pour la formulation de produits phytosanitaires.

9. Procédé de préparation de produits suivant la revendication 8, **caractérisé en ce qu'**on mélange une quantité efficace du point de vue herbicide d'au moins un dérivé de thiényloxy-pyridine substituée par du pyrazolyle de la formule I ou d'un sel utilisable en agriculture de I suivant les revendications 1 à 3 et des adjuvants courants pour la formulation de produits phytosanitaires.

10. Procédé pour lutter contre la croissance non souhaitée de plantes, **caractérisé en ce qu'**on fait agir sur des plantes, leur espace vital et/ou des semences une quantité efficace du point de vue herbicide d'au moins un dérivé de thiényloxy-pyridine substituée par du pyrazolyle de la formule I ou d'un sel utilisable en agriculture de I suivant les revendications 1 à 3.

11. Utilisation de dérivés de thiényloxy-pyridine substituée par du pyrazolyle de la formule I et de leurs sels utilisables en agriculture suivant l'une des revendications 1 à 3, comme herbicides.
